(19) Europäisches Patentamt
European Patent Office
Office européen des brevets

(11) EP 2 655 474 B1

## (12) EUROPÄISCHE PATENTSCHRIFT

(45) Veröffentlichungstag und Bekanntmachung des Hinweises auf die Patenterteilung:
**08.10.2014 Patentblatt 2014/41**

(21) Anmeldenummer: **11797257.0**

(22) Anmeldetag: **19.12.2011**

(51) Int Cl.:
***C08G 65/00*** *(2006.01)*

(86) Internationale Anmeldenummer:
**PCT/EP2011/073164**

(87) Internationale Veröffentlichungsnummer:
**WO 2012/084762 (28.06.2012 Gazette 2012/26)**

## (54) VERFAHREN ZUR HERSTELLUNG VON POLYETHERPOLYOLEN

METHOD FOR PRODUCING POLYETHER POLYOLS

PROCÉDÉ DE PRODUCTION DE POLY-ÉTHER POLYOLS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2010 EP 10195882**

(43) Veröffentlichungstag der Anmeldung:
**30.10.2013 Patentblatt 2013/44**

(73) Patentinhaber: **Bayer Intellectual Property GmbH 40789 Monheim am Rhein (DE)**

(72) Erfinder:
• **LORENZ, Klaus**
**41539 Dormagen (DE)**
• **HOFMANN, Jörg**
**47800 Krefeld (DE)**

(74) Vertreter: **BIP Patents
c/o Bayer Intellectual Property GmbH
Creative Campus Monheim
Alfred-Nobel-Straße 10
40789 Monheim (DE)**

(56) Entgegenhaltungen:
EP-A1- 1 528 073        EP-A1- 1 577 334
EP-A1- 2 028 211        WO-A1-01/53381
WO-A1-2009/152954       WO-A2-2006/094979

## Beschreibung

Gegenstände der vorliegenden Erfindung sind ein aufarbeitungsfreies Verfahren zur Herstellung von Polyetherpolyolen, die durch das aufarbeitungsfreie Verfahren erhältlichen Polyetherpolyole sowie die Verwendung der erfindungsgemäßen Polyetherpolyole zur Herstellung von Polyurethanen.

Für die Herstellung von Polyurethanen geeignete Polyetherpolyole können über verschiedene Herstellverfahren erhalten werden. Von großtechnischer Bedeutung ist zum Einen die basisch katalysierte Anlagerung von Alkylenoxiden an H-funktionelle Starterverbindungen, zum anderen die Verwendung von Doppelmetallcyanidverbindungen als Katalysatoren ("DMC-Katalysatoren") für die Anlagerung von Alkylenoxiden an H-funktionelle Starterverbindungen. Die durch (Lewis-)Säuren katalysierte Anlagerung von Alkylenoxiden an geeignete Starterverbindungen ist von untergeordneter Bedeutung.

Unter Alkalimetallhydroxidkatalyse nehmen bei steigender Molmasse des Polymerisates unerwünschte Nebenreaktionen deutlich zu. Insbesondere ist hier die Isomerisierung von Propylenoxid zu Allylalkohol zu nennen, die bei hohen Äquivalentgewichten (bzw. niedrigen OH-Zahlen) zu einem hohen Anteil monofunktioneller Polyetherspezies im Reaktionsgemisch führt. Die monofunktionellen Polyethermoleküle wirken sich nachteilig auf das Durchhärteverhalten und das physikalische Eigenschaftsprofil von Polyurethansystemen aus.

Durch den Einsatz der DMC-Katalysatoren ist es möglich geworden, die Addition von Alkylenoxiden, insbesondere Propylenoxid, an H-funktionelle Starterverbindungen bis zu sehr niedrigen OH-Zahlen voranzutreiben, ohne dass die oben erwähnte Isomerisierung von Propylenoxid zu Allylalkohol in nennenswertem Maße eintritt. Hochaktive DMC-Katalysatoren, die z.B. in US-A 5470813, EP-A 700949, EP-A 743093, EP-A 761708, WO-A 97/40086, WO-A 98/16310 und WO-A 00/47649 beschrieben sind, besitzen zudem eine außerordentlich große Aktivität und ermöglichen die Polyetherpolyolherstellung bei sehr geringen Katalysatorkonzentrationen (25 ppm oder weniger), so dass eine Abtrennung des Katalysators aus dem fertigen Produkt nicht mehr erforderlich ist. Ein typisches Beispiel sind die in EP-A 700949 beschriebenen hochaktiven DMC-Katalysatoren, die neben einer Doppelmetallcyanid-Verbindung (z.B. Zinkhexacyanocobaltat(III)) und einem organischen Komplexliganden (z.B. tert.-Butanol) noch ein Polyetherpolyol mit einer zahlenmittleren Molmasse größer als 500 g/mol enthalten.

Ein Charakteristikum von DMC-Katalysatoren ist ihre ausgeprägte Empfindlichkeit gegen hohe Konzentrationen an Hydroxylgruppen, welche beispielsweise durch große Mengen an Startern wie Ethylenglykol, Propylenglykol, Glycerin, Trimethylolpropan, Sorbitol oder Saccharose hervorgerufen werden, und polare Verunreinigungen des Reaktionsgemisches. Die DMC-Katalysatoren können dann während der Reaktionsinitiierungsphase nicht in die polymerisationsaktive Form überführt werden oder bereits laufende Alkylenoxidadditionsreaktionen können durch die kontinuierliche Zufuhr hoher Konzentrationen an Hydroxylgruppen sowie polarer Verunreinigungen zum Erliegen kommen. Verunreinigungen können beispielsweise Wasser, Verbindungen mit einer hohen Zahl in enger Nachbarschaft stehender Hydroxylgruppen wie Kohlenhydrate und Kohlenhydratderivate oder Verbindungen mit basischen Gruppen wie beispielsweise Amine sein. Auch Substanzen mit in enger Nachbarschaft stehenden Carbonylgruppen bzw. zu Hydroxylgruppen benachbarten Carbonylgruppen wirken sich nachteilig auf die Katalysatoraktivität aus. Um Starter mit hohen Konzentrationen an OH-Gruppen, bzw. Starter mit als Katalysatorgiften anzusehenden Verunreinigungen dennoch DMC-katalysierten Alkylenoxidadditionsreaktionen unterziehen zu können, müssen die Hydroxylgruppenkonzentration gesenkt bzw. die Katalysatorgifte unschädlich gemacht werden. Hierzu können aus diesen Starterverbindungen mittels basischer Katalyse zunächst Vorpolymerisate hergestellt werden, welche dann nach Aufarbeitung mittels DMC-Katalyse in die erwünschten Alkylenoxidadditionsprodukte hoher Molmasse überführt werden. Nachteilig bei dieser Vorgehensweise ist, dass das mittels basischer Katalyse erhaltene Vorpolymerisat sehr sorgfältig aufgearbeitet werden muss, um die Deaktivierung des DMC-Katalysators durch über das Vorpolymerisat eingeschleppte basische Katalysatorspuren auszuschließen.

Dieser Nachteil kann durch das Verfahren der kontinuierlichen Starterdosierung, welches in WO-A 97/29146 offenbart ist, überwunden werden. Hierbei werden kritische Starterverbindungen im Reaktor nicht vorgelegt, sondern neben den Alkylenoxiden dem Reaktor während der Reaktion kontinuierlich zugeführt. Als Startmedium für die Reaktion können in diesem Verfahren Vorpolymerisate vorgelegt werden, auch ist die Verwendung kleiner Mengen des herzustellenden Produktes selbst als Startmedium möglich. Die Notwendigkeit, für weitere Alkylenoxidadditionen geeignete Vorpolymerisate zunächst separat herstellen zu müssen, entfällt somit.

Ebenfalls können Polyetherpolyole aufarbeitungsfrei nach einem Verfahren, wie es in WO-A 98/03571 beschrieben ist, vollkontinuierlich hergestellt werden. Hierbei werden neben einem oder mehreren Alkylenoxiden und einem oder mehreren Startern auch der DMC-Katalysator dem Reaktor bzw. einem Reaktorsystem unter Alkoxylierungsbedingungen kontinuierlich zugeführt und das Produkt kontinuierlich dem Reaktor bzw. dem Reaktorsystem nach einer vorwählbaren mittleren Verweilzeit entnommen.

Sowohl das Verfahren der kontinuierlichen Starterzudosierung als auch das vollkontinuierliche Polyetherpolyolherstellverfahren besitzen den Nachteil, dass Polyether mit Blockstrukturen, insbesondere solche mit kurzen Innenblöcken, nur unter großen Schwierigkeiten herstellbar sind: Beim Verfahren der kontinuierlichen Starterzudosierung muss die Starterzudosierung vor Ende der Dosierung des ersten Alkylenoxidblocks bereits abgeschlossen sein, um Produkte mit homogen verteilten Blocklängen zu erhalten. Dies ist insbesondere dann schwierig, wenn innere Blöcke mit Blockäquivalentmolmassen von 53 Da bis 350 Da angestrebt werden, da es dann notwendig wird, das Verhältnis von Starter zu Alkylenoxid im zudosierten Eduktstrom so zu erhöhen, dass wiederum die Gefahr besteht, kritische Konzentrationen an Hydroxylgruppen sowie polaren Verunreinigungen zu erreichen. In solchen Fällen verlieren die Katalysatoren während der Starterzudosierungsphase zunehmend an Aktivität, was sich z.B. durch einen Druckanstieg im Reaktor infolge steigender Konzentration an freiem Alkylenoxid bemerkbar macht. Beim vollkontinuierlichen Polyetherpolyolherstellverfahren müssen für Produkte mit Blockstrukturen aufwändige, kontinuierlich durchströmte Reaktorkaskaden und Verweilzeitstrecken installiert werden. Sowohl das kontinuierliche Starterzudosierungsverfahren als auch das vollkontinuierliche Verfahren sind außerdem nur schlecht geeignet, hochschmelzende oder sich unterhalb des Schmelzpunktes zersetzende Starterverbindungen wie beispielsweise Zucker, Sorbit oder Pentaerythrit aufarbeitungsfrei in langkettige Polyole zu überführen. Die Dosierung solcher Starter muss über aufwändig beheizte Dosierstrecken oder in Lösung erfolgen.

Unter Äquivalentmolmasse ist die durch die Zahl der aktiven Wasserstoffatome geteilte Gesamtmasse des aktive Wasserstoffatome enthaltenden Materials zu verstehen. Im Falle von hydroxygruppenhaltigen Materialien wird sie berechnet durch folgende Formel:

$$\text{Äquivalentmolmasse} = 56100 \,/\, \text{OH-Zahl [mg KOH/g]}$$

Die OH-Zahl kann z. B. titrimetrisch nach der Vorschrift der DIN 53240 oder spektroskopisch über NIR bestimmt werden.

In EP-A 0090445 wird der Zusatz von Katalysator-"Promotern" zur Anhebung der Aktivität von DMC-Katalysatoren älterer Generation beansprucht. Solche "Promoter" sind Salze aus mindestens zweiwertigen Metallkationen und metallfreien Anionen und/oder metallfreie Säuren. Die "Promoter" werden der Katalysator/Startermischung separat zugesetzt. Es wird betont, dass die Abwesenheit von Alkalimetallsalzen essentiell ist, da diese die Aktivität von DMC-Katalysatoren mindern. Vor diesem Hintergrund ist die vorliegende Erfindung besonders überraschend.

In EP-A 1400281 werden salzhaltige, insbesondere alkalimetallhalogenidhaltige DMC-Katalysatoren beansprucht, die zu Polyethern mit reduziertem Gehalt an hochmolekularen Verunreinigungen führen. In der vorliegenden Erfindung erwiesen sich kaliumchloridhaltige Starter jedoch als völlig ungeeignet, da keinerlei Katalysatoraktivierung beobachtet wurde. In EP-A 1577334 werden bevorzugt mit Phosphorsäure acidifizierte Starter in DMC-katalysierten Alkylenoxidadditionsprozessen mit kontinuierlicher Starterzudosierung beansprucht, die zu erhöhter Katalysatorlebensdauer führen, wenn relativ kurzkettige Polyether über DMC-Katalyse hergestellt werden sollen, also verhältnismäßig hohe Starter/Alkylen-oxidverhältnisse während der Dosierphase vorliegen. Der Zusatz von (sauren) Salzen wird nicht erwähnt. Im Rahmen der vorliegenden Erfindung durchgeführte Arbeiten zeigen, dass phosphathaltige Starterverbindungen die Aktivierung von DMC-Katalysatoren verhindern. In WO-A 99/14258 werden ebenfalls acidifizierte Starter in DMC-katalysierten Alkylenoxidadditionsprozessen mit kontinuierlicher Starterzudosierung beansprucht. Wiederum wird Phosphorsäure als besonders bevorzugte Säure hervorgehoben. Salze der Schwefelsäure werden nicht erwähnt.

US-B 6642423 beansprucht ein Verfahren zum Erhalt von Polyethern mit ethylenoxidhaltigen Innenblöcken. Diese können einstufig direkt durch DMC-katalysierte Ethylenoxidaddition an niedermolekulare Starterverbindungen wie Glycerin gefolgt von einem Propylenoxidblock oder einem propylenoxidreichen Block erhalten werden. Das Verfahren nutzt nicht den vorteilhaften Effekt der Gegenwart eines Schwefelsäuresalzes auf die Unterdrückung der Bildung hochmolekularer Verunreinigungen und ist außerdem sehr teuer, da sich DMC-Katalysatoren im Kontakt mit niedermolekularen Starterverbindungen wie Glycerin nur in sehr hohen Konzentrationen aktivieren lassen.

In EP-A 1528073 wird die zweistufige Herstellung von typischen langkettigen Polyolen mit Ethylenoxidendblock in einem Reaktor beansprucht. Die aus dem jeweils vorhergehenden Ansatz resultierende Restalkalinität wird vor oder während der Dosierung des Starters und des DMC-Katalysators für den nachfolgenden Ansatz durch Zugabe einer Säure, die ein im langkettigen Polyol mit Ethylenoxidendblock lösliches Salz bildet, entfernt. Hierzu ist im Allgemeinen nur eine Alkylbenzolsulfonsäure geeignet, da die im Reaktor verbliebenen Polyolreste hohe Äquivalentmolmassen aufweisen und Salze rein anorganischer Säuren nicht zu lösen in der Lage sind. Nachteilig beim Einsatz von Alkylbenzolsulfonsäuren sind die hohen Kosten, die zum einen durch die hohen Säurepreise, zum anderen durch die relativ hohen Molmassen der Säuren verursacht werden. Außerdem erfordert der beanspruchte Prozess immer noch einen Aufarbeitungsschritt wie Destillation/Filtration oder Ionenaustausch, bei dem die großen auf herkömmliche Weise erhaltenen Salzmengen entfernt werden.

WO-A 2006/094979 beansprucht ein vereinfachtes Verfahren zur Herstellung von DMC-Katalysatoren, bei dem durch die Gegenwart von starken Mineralsäuren während der Katalysatorfällung die Cyanometallatsäure in situ erzeugt wird. Bei den so hergestellten Katalysatoren handelt es sich um klassische DMC-Katalysatoren, mit denen allein sich kein aufarbeitungsfreies Eintopfverfahren zur Herstellung langkettiger Blockcopolyether mit Innenblöcken mit Blockäquivalentmolmassen von 53 Da bis 350 Da realisieren lässt.

In WO-A 01/53381 werden Kombinationen aus Lewis- oder Bronstedsäuren und DMC-Katalysatoren verwendet, die zu verkürzten Induktionsperioden beim Anfahren der Alkylenoxidadditionsreaktion führen sollen. Durch die Kombination bestimmter Säuren/saurer Salze mit DMC-Katalysatoren erzielbare synergistische Effekte auf die Polyetherqualität sind nicht herausgearbeitet worden, die analytischen Daten der erhaltenen Polyetherpolyole, insbesondere die erhöhten Gehalte an primären Hydroxygruppen, deuten vielmehr schlicht auf parallel ablaufende säure- bzw. DMC-katalysierte Alkylenoxidadditionsreaktionen hin.

In EP-A 1073689 wird die Herstellung von Polyolvorstufen mit OH-Zahlen von 100 bis 150 mg KOH/g unter lewissauren Bedingungen gefolgt von einem DMC-katalysierten Propylenoxidadditionsschritt beansprucht. Eine Abtrennung der lewissauren Katalysatoren, im Wesentlichen Perfluoralkylsulfonsäuresalze der Lanthaniden, aus der Vorstufe vor dem DMC-Schritt erfolgt nicht. Es handelt sich somit um ein aufarbeitungsfreies Eintopfverfahren, bei dem jedoch die in EP-A 0855417 beschriebene auffällige Neigung der Lewis-sauren Katalysatoren zur Bildung flüchtiger Nebenprodukte und deren hohe Kosten als nachteilig einzustufen sind.

WO-A 2007/082596 lehrt die Herstellung von mit Alkali- bzw. Ammoniumsalzen modifizierten DMC-Katalysatoren, die sich durch erhöhte Aktivitäten auszeichnen. Mit dem in WO-A 2007/082596 offenbarten Verfahren ist die Durchführung eines aufarbeitungsfreien Verfahrens ausgehend von niedermolekularen Starterverbindungen nicht möglich. Die positiven Effekte schwefelsäuresalzhaltiger Starter in Bezug auf die Bildung hochmolekularer Verunreinigungen lassen sich nach der Lehre der WO 2007/082596 nicht erzielen.

Die Aufgabe bestand daher darin, ein aufarbeitungsfreies Verfahren für die Herstellung von Polyetherpolyolen bereitzustellen, das sich durch eine geringe Neigung zur Bildung hochmolekularer Verunreinigungen auszeichnet. Das erfindungsgemäße Verfahren sollte vorzugsweise auch geeignet sein, Polyetherpolyole mit besonders hydrophilen Innenblöcken zugänglich zu machen. Darüber hinaus sollen die auf den erfindungsgemäßen Polyetherpolyolen basierenden Polyurethan-Weichschaumstoffe eine höhere Stauchhärte aufweisen im Vergleich zu Weichschaumstoffen, die nur auf Füllstoff-freien Polyetherpoyololen gemäß dem Stand der Technik basieren.

Überraschenderweise wurde gefunden, dass die oben genannte Aufgabe gelöst wird durch ein Verfahren für die Herstellung von Polyetherpolyolen (1) mit einer OH-Zahl von 3 mg KOH/g bis 150 mg KOH/g, bevorzugt 10 mg KOH/g bis 60 mg KOH/mg besonders bevorzugt 20 mg KOH/g bis 50 mg KOH/g, dadurch gekennzeichnet, dass

(i)

(i-1) eine H-funktionelle Starterverbindung A1.1) mit einem oder mehreren Alkylenoxiden A1.2) in Anwesenheit eines basischen Katalysators umgesetzt wird unter Erhalt eines Alkoxylates mit einer Äquivalentmolmasse von 53 Da bis 350 Da, und anschließend

(i-2) die Komponente A1) mit Schwefelsäure neutralisiert wird, wobei die Neutralisation der alkalischen polymerisationsaktiven Zentren des rohen Alkylenoxidadditionsproduktes durch Zugabe von Schwefelsäure derart durchgeführt wird, dass von 66 mol-% bis 100 mol-% der eingesetzten Säure nur die erste Dissoziationsstufe zur Neutralisation der im Rohpolymerisat enthaltenden Katalysatormenge wirksam wird, und wobei auf die Abtrennung der gebildeten Salze verzichtet wird, unter Erhalt der Komponente A) und

(ii) anschließend die Komponente A) umgesetzt wird mit einem oder mehreren Alkylenoxiden B1) in Anwesenheit eines DMC-Katalysators B2).

Weitere Gegenstände der vorliegenden Erfindung sind Polyetherpolyole enthaltend ein saures Schwefelsäuresalz, erhältlich nach dem erfindungsgemäßen Verfahren, die Verwendung dieser Polyetherpolyole zur Herstellung von Polyurethanen und Polyurethane enthaltend die erfindungsgemäßen Polyetherpolyole.

In einer Ausführungsform des erfindungsgemäßen Verfahrens werden saure Schwefelsäuresalze A2) dem Polyetherpolyol A1) separat zugesetzt und die so erhältliche Komponente A) wird dann in Schritt ii) weiter umgesetzt. A2) wird A1) in Mengen von 95 bis 12000 ppm, bevorzugt in Mengen von 95 bis 2400 ppm und besonders bevorzugt in Mengen 95 bis 1700 ppm, bezogen auf die Menge A1), zugesetzt.

Unter den sauren Schwefelsäuresalzen A2) sind Hydrogensulfate A2.1) und Sulfate A2.2) zu verstehen, wobei das Gewichtsverhältnis von A2.1) : A2.2) 50 bis 100 : 50 bis 0 beträgt.

Hydrogensulfate A2.1) sind

Alkalimetall- (d.h. Li, Na, K, Rb, Cs),

Erdalkalimetall- (d.h. Be, Ca, Mg, Sr, Ba), oder

Ammoniumhydrogensulfate der allgemeinen Formel $[NR^1R^2R^3H]^+[HSO_4]^-$, wobei

$R^1R^2R^3$ unabhängig voneinander H, $C_1$-$C_{20}$-Alkyl (z.B. Methyl, Ethyl, Propyl, Butyl), $C_5$-$C_{20}$-Cycloalkyl (z.B. Cyclopentyl, Cyclohexyl), $C_6$-$C_{20}$ Aryl (z.B Phenyl) sein können, und wobei die Reste $R^1$, $R^2$ und/oder $R^3$ auch miteinander so verbunden sein können, dass ein cyclisches Ammoniumion entsteht, wie besipielsweise Piperazinium, Imidazolinium, Pyridinium, Morpholinium, und

Sulfate A2.2) sind

Alkalimetall- (d.h. Li, Na, K, Rb, Cs),

Erdalkalimetall- (d.h. Be, Ca, Mg, Sr, Ba), oder

Ammoniumsulfate der allgemeinen Formel $[NR^1R^2R^3H]^+[HSO_4]^-$, wobei $R^1R^2R^3$ unabhängig voneinander H, $C_1$-$C_{20}$-Alkyl (z.B. Methyl, Ethyl, Propyl, Butyl), $C_5$-$C_{20}$-Cycloalkyl (z.B. Cyclopentyl, Cyclohexyl), $C_6$-$C_{20}$ Aryl (z.B Phenyl) sein können, und wobei die Reste $R^1$, $R^2$ und/oder $R^3$ auch miteinander so verbunden sein können, dass ein cyclisches Ammoniumion entsteht, wie beispielsweise Piperazinium, Imidazolinium, Pyridinium, Morpholinium.

Bevorzugt werden als Hydrogensulfate A2.1) Alkalimetallhydrogensulfate, ganz besonders bevorzugt Kaliumhydrogensulfat und als Sulfate A2.2) Alkalimetallsulfate und ganz besonders bevorzugt Kaliumsulfat im erfindungsgemäßen Verfahren eingesetzt.

In einer bevorzugten Ausführungsform des erfindungsgemäßen Verfahrens, wird die Komponente A2) während des Verfahrens durch Neutralisation des Polyetherpolyol A1) mit Schwefelsäure unter Bildung der Komponente A) erzeugt und ohne Filtrationsschritt direkt in Schritt (ii) unter DMC-Katalyse mit einem oder mehreren Alkylenoxiden B1) zu den erfindungsgemäßen Polyetherpolyolen (1) umgesetzt. In dieser bevorzugten Ausführungsform des erfindungsgemäßen Verfahrens wird daher Komponente A) hergestellt durch die Schritte

(i-1) Umsetzung einer H-funktionellen Starterverbindung A1.1) mit einem oder mehreren Alkylenoxiden A1.2) in Anwesenheit eines basischen Katalysators, bis die Komponente A1) Äquivalentmolmassen von 53 Da bis 350 Da erreicht, und anschließend

(i-2) die Komponente A1) mit Schwefelsäure neutralisiet wird, wobei die Neutralisation der alkalischen polymerisationsaktiven Zentren des rohen Alkylenoxidadditionsproduktes durch Zugabe von Schwefelsäure derart durchgeführt wird, dass von 66 mol-% bis 100 mol-% der eingesetzten Säure nur die erste Dissoziationsstufe zur Neutralisation der im Rohpolymerisat enthaltenden Katalysatormenge wirksam wird, und wobei auf die Abtrennung der gebildeten Salze verzichtet wird, und

(i-3) gegebenenfalls die Entfernung von Reaktionswasser und mit der Säure eingebrachte Wasserspuren bei einem absoluten Druck von 1 bis 500 mbar und bei Temperaturen von 20 bis 200 °C, bevorzugt bei 80 bis 180°C.

Im Folgenden wird das erfindungsgemäße Verfahren detailliert beschrieben:

*Schritt (i), Schritte (i-1) bis (i-3):*

*(i-1)*

Die H-funktionellen Starterverbindungen (Komponente A1.1) werden in einer Ausführungsform des erfindungsgemäßen Verfahrens in Schritt (i-1) im Reaktor vorgelegt und mit dem basischen Katalysator versetzt und mit einem oder mehreren Alkylenoxiden A1.2) umgesetzt.

Als basischer Katalysator können Alkalimetallhydroxide, Alkali- und Erdalkalimetallhydride, Alkali- und Erdalkalimetallcarboxylate oder Erdalkalihydroxide verwendet werden. Alkalimetalle sind ausgewählt aus der Gruppe bestehend aus Li, Na, K, Rb, Cs und die Erdalkalimetalle sind ausgewählt aus der Gruppe bestehend aus Be, Ca, Mg, Sr, Ba. Ebenfalls können organische basische Katalysatoren wie beispielsweise Amine eingesetzt werden. Hierunter fallen aliphatische Amine oder Alkanolamine wie N,N-Dimethylbenzylamin, Dimethylaminoethanol, Dimethylaminopropanol, N-Methyldiethanolamin, Trimethylamin, Triethylamin, N,N-Dimethylcyclohexylamin, N-Methylpyrrolidin, N,N,N',N'-Tetramethylethylendiamin, Diazabicyclo[2,2,2]octan, 1,4-Dimethylpiperazin oder N-Methylmorpholin. Ebenfalls gut verwendbar sind auch aromatische Amine wie Imidazol und alkylsubstituierte Imidazolderivate, N,N-Dimethylanilin, 4-(N,N-Dimethyl)aminopyridin sowie teilweise vernetzte Copolymere aus 4-Vinylpyridin oder Vinylimidazol und Divinylbenzol. Eine umfassende Übersicht über geeignete Amine ist von M. Ionescu et al. in "Advances in Urethanes Science and Technology", 1998, 14, 151-218 gegeben worden. Bevorzugte aminische Katalysatoren sind tertiäre aliphatische Amine oder Alkanolamine sowie Imidazol und die erwähnten Imidazol- bzw. Pyridinderivate. Solche aminischen Katalysatoren können in Konzentrationen, bezogen auf die erhaltene Produktmenge A1), von 200 ppm bis 10000 ppm eingesetzt werden, bevorzugt ist der Konzentrationsbereich von 200 ppm bis 5000 ppm. Bevorzugte anorganische basische Katalysatoren sind die Alkalimetallhydroxide, ganz besonders bevorzugt ist Kaliumhydroxid. Ein solcher alkalimetallhaltiger Katalysator kann der H-funktionellen Starterverbindung als wässrige Lösung oder als Feststoff zugeführt werden. Die auf die erhaltene Produktmenge A1) bezogene Katalysatorkonzentration beträgt im Falle der Verwendung anorganischer basischer Katalysatoren 40 ppm bis 5000 ppm, bevorzugt 40 ppm bis 1000 ppm, besonders bevorzugt 40 ppm bis 700 ppm. Das Lösungswasser und/oder das bei der Reaktion der aktiven Wasserstoffatome mit dem Katalysator freigesetzte Wasser kann vor Beginn der Dosierung eines oder mehreren Alkylenoxide im Vakuum bei einem absoluten Druck von 1 bis 500 mbar bei Temperaturen von 20 bis 200 °C, bevorzugt bei 80 bis 180 °C, entfernt werden.

Als basische Katalysatoren können auch vorgefertigte Alkylenoxid-Additionsprodukte von H-funktionellen Starterverbindungen mit Alkoxylatgehalten von 0,05 bis 50 Äquivalenz-% eingesetzt werden, sogenannte "polymere Alkoxylate". Unter dem Alkoxylatgehalt des Katalysators ist der durch eine Base, üblicherweise ein Alkalimetallhydroxid, durch Deprotonierung entfernte Anteil aktiver Wasserstoffatome an allen ursprünglich im Alkylenoxid-Additionsprodukt des Katalysators vorhanden gewesenen aktiven Wasserstoffatomen zu verstehen. Die Einsatzmenge des polymeren Alkoxylates richtet sich natürlich nach der für die Produktmenge A1) angestrebten Katalysatorkonzentration, wie im vorangehenden Abschnitt beschrieben.

H-funktionelle Starterverbindungen sind solche Verbindungen, die mindestens ein Zerewitinoff-aktives Wasserstoffatom, manchmal auch nur als "aktiver Wasserstoff" bezeichnet, enthalten. Ein an N, O, oder S gebundener Wasserstoff wird als Zerewitinoffaktiver Wasserstoff bezeichnet, wenn er nach einem von Zerewitinoff aufgefundenen Verfahren durch Umsetzung mit Methylmagnesiumjodid Methan liefert. Typische Beispiele für Verbindungen mit Zerewitinoff-aktivem Wasserstoff sind Verbindungen, die Carboxyl-, Hydroxyl-, Amino-, Imino- oder Thiol-Gruppen als funktionelle Gruppen enthalten. Geeignete H-funktionelle Starterverbindungen weisen meist Funktionalitäten von 1 bis 35, bevorzugt von 1 bis 8 auf. Ihre Molmassen betragen von 17 g/mol bis 1200 g/mol. Neben den bevorzugt zu verwendenden hydroxyfunktionellen Startern können auch aminofunktionelle Starter eingesetzt werden. Beispiele für hydroxyfunktionelle Starterverbindungen sind Methanol, Ethanol, 1-Propanol, 2-Propanol und höhere aliphatische Monole, insbesondere Fettalkohole, Phenol, alkylsubstituierte Phenole, Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit, Sorbit, Saccharose, Hydrochinon, Brenzcatechin, Resorcin, Bisphenol F, Bisphenol A, 1,3,5-Trihydroxybenzol, sowie methylolgruppenhaltige Kondensate aus Formaldehyd und Phenol oder Harnstoff. Es können auch hochfunktionelle Starterverbindungen auf Basis von hydrierten Stärkehydrolyseprodukten eingesetzt werden. Solche sind beispielsweise in EP-A 1525244 beschrieben. Beispiele für geeignete aminogruppenhaltige H-funktionelle Starterverbindungen sind Ammoniak, Ethanolamin, Diethanolamin, Triethanolamin, Isopropanolamin, Diisopropanolamin, Ethylendiamin, Hexamethylendiamin, Anilin, die Isomere des Toluidins, die Isomere des Diaminotoluols, die Isomere des Diaminodiphenylmethans sowie bei der Kondensation von Anilin mit Formaldehyd zu Diaminodiphenylmethan anfallende höherkernige Produkte, ferner methylolgruppenhaltige Kondensate aus Formaldehyd und Melamin sowie Mannichbasen. Außerdem können als Starterverbindungen auch Ringöffnungsprodukte aus cyclischen Carbonsäureanhydriden und Polyolen eingesetzt werden. Beispiele sind Ringöffnungsprodukte aus Phthalsäureanhydrid, Bernsteinsäureanhydrid, Maleinsäureanhydrid einerseits und Ethylenglykol, Diethylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit oder Sorbit andererseits. Daneben ist es auch möglich, ein- oder mehrfunktionelle Carbonsäuren direkt als Starterverbindungen einzusetzen.

Ferner können dem Prozess auch vorgefertigte Alkylenoxidadditionsprodukte der erwähnten Starterverbindungen, also Polyetherpolyole vorzugsweise mit OH-Zahlen von 160 bis 1000 mg KOH/g, bevorzugt 250 bis 1000 mg KOH/g, zugesetzt werden. Auch ist es möglich, im erfindungsgemäßen Prozess Polyesterpolyole vorzugsweise mit OH-Zahlen im Bereich von 6 bis 800 mg KOH/g als Co-Starter mit dem Ziel der Polyetheresterherstellung einzusetzen. Hierfür geeignete Polyesterpolyole können beispielsweise aus organischen Dicarbonsäuren mit 2 bis 12 Kohlenstoffatomen und mehrwertigen Alkoholen, vorzugsweise Diolen, mit 2 bis 12 Kohlenstoffatomen, vorzugsweise 2 bis 6 Kohlenstoffatomen nach bekannten Verfahren hergestellt werden.

Des Weiteren können als H-funktionelle Startersubstanzen Polycarbonatpolyole, Polyestercarbonatpolyole oder Polyethercarbonatpolyole, bevorzugt Polycarbonatdiole, Polyestercarbonatdiole oder Polyethercarbonatdiole vorzugsweise jeweils mit OH-Zahlen im Bereich von 6 bis 800 mg KOH/g, als Co-Starter verwendet werden. Diese werden beispielsweise durch Umsetzung von Phosgen, Dimethylcarbonat, Diethylcarbonat oder Diphenylcarbonat mit di- oder höherfunktionellen Alkoholen oder Polyesterpolyolen oder Polyetherpolyolen hergestellt.

Im erfindungsgemäßen Verfahren dienen bevorzugt aminogruppenfreie H-funktionelle Starterverbindungen mit Hydroxygruppen als Träger der aktiven Wasserstoffe wie beispielsweise Methanol, Ethanol, 1-Propanol, 2-Propanol und höhere aliphatische Monole, insbesondere Fettalkohole, Phenol, alkylsubstituierte Phenole, Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit, Sorbit, Saccharose, Hydrochinon, Brenzcatechin, Resorcin, Bisphenol F, Bisphenol A, 1,3,5-Trihydroxybenzol, methylolgruppenhaltige Kondensate aus Formaldehyd und Phenol und hydrierte Stärkehydrolyseprodukte. Es können auch Gemische verschiedener H-funktioneller Starterverbindungen eingesetzt werden.

Die im Reaktor gemeinsam mit dem basischen Katalysator vorgelegten H-funktionellen Starterverbindungen A1.1) werden in Schritt (i-1) unter Inertgasatmosphäre bei Temperaturen von 80 bis 180 °C, bevorzugt bei 100 bis 170 °C mit einem oder mehreren Alkylenoxiden A1.2) zur Reaktion gebracht, wobei die Alkylenoxide in der gängigen Weise dem Reaktor kontinuierlich derart zugeführt werden, dass die sicherheitstechnischen Druckgrenzen des verwendeten Reaktorsystems nicht überschritten werden. Insbesondere bei der Dosierung von ethylenoxidhaltigen Alkylenoxidgemischen oder reinem Ethylenoxid ist darauf zu achten, dass ein ausreichender Inertgaspartialdruck im Reaktor während der Anfahr- und Dosierphase aufrechterhalten wird. Dieser kann beispielsweise durch Edelgase oder Stickstoff eingestellt werden. Die Reaktionstemperatur kann während der Alkylenoxiddosierphase natürlich innerhalb der beschriebenen Grenzen variiert werden: Es ist von Vorteil empfindliche H-funktionelle Starterverbindungen, wie beispielsweise Saccharose, zunächst bei niedrigen Reaktionstemperaturen zu alkoxylieren, und erst bei hinreichendem Starterumsatz zu höheren Reaktionstemperaturen überzugehen. Alkylenoxide können dem Reaktor auf unterschiedliche Weise zugeführt werden: Möglich ist eine Dosierung in die Gasphase oder direkt in die Flüssigphase, z. B. über ein Tauchrohr oder einen in der Nähe des Reaktorbodens in einer gut durchmischten Zone befindlichen Verteilerring. Bei Dosierung in die Flüssigphase sollten die Dosieraggregate selbstleerend ausgelegt sein, beispielsweise durch Anbringen der Dosierbohrungen an der Unterseite des Verteilerrings. Generell sollte durch apparative Maßnahmen, beispielsweise durch die Montage von Rückschlagklappen, ein Rückströmen von Reaktionsmedium in die Dosieraggregate verhindert werden. Wird ein Alkylenoxidgemisch dosiert, können die jeweiligen Alkylenoxide dem Reaktor separat oder als Mischung zugeführt werden. Eine Vorvermischung der Alkylenoxide kann beispielsweise durch ein in der gemeinsamen Dosierstrecke befindliches Mischaggregat erreicht werden ("inline-blending"). Es hat sich auch bewährt, Alkylenoxide pumpendruckseitig in einen beispielsweise über Wärmetauscher geführten Umpumpkreislauf einzeln oder vorgemischt zu dosieren. Für die gute Durchmischung mit dem Reaktionsmedium ist es dann von Vorteil ein hochscherendes Mischaggregat in den Alkylenoxid-/Reaktionsmediumstrom zu integrieren. Die Temperatur der exothermen Alkylenoxidadditionsreaktion wird durch Kühlung auf dem gewünschten Niveau gehalten. Gemäß dem Stand der Technik zur Auslegung von Polymerisationsreaktoren für exotherme Reaktionen (z.B. Ullmann's Encyclopedia of Industrial Chemistry, Vol. B4, pp 167ff, 5th Ed., 1992) erfolgt eine solche Kühlung im Allgemeinen über die Reaktorwand (z.B. Doppelmantel, Halbrohrschlange) sowie mittels weiterer intern im Reaktor und/oder extern im Umpumpkreislauf angeordneter Wärmetauscherflächen, z.B. an Kühlschlangen, Kühlkerzen, Platten- Rohrbündel- oder Mischerwärmetauschern Diese sollten so ausgelegt sein, dass auch zu Beginn der Dosierphase, d. h. bei kleinem Füllstand, effektiv gekühlt werden kann.

Generell sollte in allen Reaktionsphasen durch Auslegung und Einsatz handelsüblicher Rührorgane für eine gute Durchmischung des Reaktorinhaltes gesorgt werden, wobei hier insbesondere ein- oder mehrstufig angeordnete Rührer oder großflächig über die Füllhöhe wirkende Rührertypen geeignet sind (siehe z. B. Handbuch Apparate; Vulkan-Verlag Essen, 1. Aufl. (1990), S.188 - 208). Technisch besonders relevant ist hierbei eine im Mittel über den gesamten Reaktorinhalt eingetragene Mischenergie, die im Allgemeinen im Bereich von 0,2 bis 5 W/1 liegt, mit entsprechend höheren lokalen Leistungseinträgen im Bereich der Rührorgane selbst und ggf. bei niedrigeren Füllständen. Um eine optimale Rührwirkung zu erzielen, können im Reaktor gemäß allgemeinem Stand der Technik Kombinationen aus Stromstörern (z. B. Flach- oder Rohrstromstörer) und Kühlschlangen (oder Kühlkerzen) angeordnet werden, die sich auch über den Behälterboden erstrecken können. Die Rührleistung des Mischaggregates kann während der Dosierphase auch füllstandsabhängig variiert werden, um in kritischen Reaktionsphasen einen besonders hohen Energieeintrag zu gewährleisten. Beispielsweise kann es vorteilhaft sein, feststoffhaltige Dispersionen, die zu Reaktionsbeginn beispielsweise bei der Verwendung von Saccharose vorliegen können, besonders intensiv zu durchmischen. Außerdem sollte insbesondere beim Einsatz fester H-funktioneller Starterverbindungen durch die Wahl des Rühraggregates sichergestellt werden, dass eine ausreichende Dispergierung des Feststoffes im Reaktionsgemisch gewährleistet ist. Bevorzugt werden hier bodengängige Rührstufen sowie besonders zur Suspendierung geeignete Rührorgane eingesetzt. Ferner sollte die Rührergeometrie zur Minderung des Aufschäumens von Reaktionsprodukten beitragen. Das Aufschäumen von Reaktionsgemischen kann beispielsweise nach Ende der Dosier- und Nachreaktionsphase beobachtet werden, wenn Restalkylenoxide zusätzlich im Vakuum bei absoluten Drücken im Bereich von 1 bis 500 mbar entfernt werden. Für solche Fälle haben sich Rührorgane als geeignet herausgestellt, die eine kontinuierliche Durchmischung der Flüssigkeitsoberfläche erzielen. Je nach Anforderung weist die Rührwelle ein Bodenlager und gegebenenfalls weitere Stützlager im Behälter auf. Der Antrieb der Rührerwelle kann dabei von oben oder von unten erfolgen (mit zentrischer oder exzentrischer Anordnung der Welle).

Alternativ ist es auch möglich, die notwendige Durchmischung ausschließlich über einen Wärmetauscher geführten Umpumpkreislauf zu erzielen oder diesen zusätzlich zum Rühraggregat als weitere Mischkomponente zu betreiben, wobei der Reaktorinhalt nach Bedarf (typischerweise 1 bis 50 mal pro Stunde) umgepumpt wird.

Für die Durchführung des erfindungsgemäßen Verfahrens sind die unterschiedlichsten Reaktortypen geeignet. Vorzugsweise werden zylinderförmige Behälter eingesetzt, welche ein Höhen-/Durchmesserverhältnis von 1:1 1 bis 10:1 besitzen. Als Reaktorböden kommen beispielsweise Kugel-, Klöpper-, Flach,- oder Konusböden in Frage.

Nach Ende der Alkylenoxiddosierphase in Schritt (i-1) kann sich eine Nachreaktionsphase anschließen, in der restliches Alkylenoxid abreagiert. Das Ende dieser Nachreaktionsphase ist erreicht, wenn kein weiterer Druckabfall im Reaktionskessel feststellbar ist. Spuren unreagierter Epoxide können nach der Reaktionsphase gegebenenfalls im Vakuum bei einem absoluten Druck von 1 bis 500 mbar entfernt werden. Das alkalische Alkylenoxid-additionsprodukt kann durch Wasser hydrolysiert werden. Dieser Hydrolyseschritt ist jedoch für die Durchführung des erfindungsgemäßen Verfahrens nicht essentiell. Die Wassermenge beträgt hierbei bis zu 15 Gew.-%, bezogen auf die Menge des alkalischen Alkylenoxidadditionsproduktes.

*(i-2)*

Die Neutralisation der alkalischen, polymerisationsaktiven Zentren des rohen, gegebenenfalls hydrolysierten Alkylenoxidadditionsproduktes A1) aus Schritt (i-1) erfolgt erfindungsgemäß in Schritt (i-2) durch Zugabe von Schwefelsäure derart, dass von 66 mol-% bis 100 mol-% der eingesetzten Säure nur die erste Dissoziationsstufe zur Neutralisation der im Rohpolymerisat enthaltenen Katalysatormenge wirksam wird. Dies kann beispielsweise dadurch erzielt werden, dass mindestens 50 % mehr Schwefelsäure als zur Neutralisation des basischen Katalysators notwendig wäre, eingesetzt wird. Da auch die 2. Dissoziationsstufe der Schwefelsäure einen ausreichenden pKa besitzt, werden beispielsweise im erfindungsgemäßen Verfahren 0,75 bis 1 mol Schwefelsäure pro mol zu neutralisierendem Katalysator ausgewählt aus der Gruppe Natriumhydroxid, Kaliumhydroxid und/oder Cäsiumhydroxid verwendet. Die Temperatur kann bei Hydrolyse und Neutralisation in weiten Bereichen variiert werden, Grenzen können hierbei durch die Korrosionsbeständigkeit der Materialien der Neutralisationskessel oder durch den Polyolaufbau gegeben sein. Sind hydrolyseempfindliche Gruppen, wie beispielsweise Estergruppen, in den Produkten zugegen, kann beispielsweise bei Raumtemperatur neutralisiert werden. In solchen Fällen empfiehlt es sich auch, auf einen vorgeschalteten, separaten Hydrolyseschritt zu verzichten. Gemäß dem erfindungsgemäßen Verfahren wird auf die Abtrennung der gebildeten Salze verzichtet.

*(i-3)*

Nach erfolgter Neutralisation können gegebenenfalls in Schritt (i-3) Wasserspuren, die durch Zugabe verdünnter Säuren eingebracht wurden, bzw. überschüssiges Hydrolysewasser im Vakuum bei einem absoluten Druck von 1 bis 500 mbar entfernt werden. Der so erhaltenen Komponente A) können bei Bedarf während oder nach der Neutralisation Alterungsschutzmittel bzw. Antioxidantien zugesetzt werden. Weitere Aufarbeitungsschritte, wie beispielsweise Filtration, sind nicht notwendig. Die Komponente A) weist Äquivalentmolmassen von 53 Da bis 350 Da auf.

*Schritt (ii)*

Der aus den Schritten (i-1) bis (i-3) erhaltenen Komponente A) wird in einer Ausführungsform des erfindungsgemäßen Verfahrens in Schritt (ii) der DMC-Katalysator zugefügt und mit einem oder mehreren Alkylenoxiden B1) umgesetzt, bis Polyetherpolyole (1) mit einer OH-Zahl von 3 mg KOH/g bis 150 mg KOH/g, bevorzugt von 10 mg KOH/g bis 60 mg KOH/g, besonders bevorzugt 20 mg KOH/g bis 50 mg KOH/g erhalten werden. Komponente A) können vor der Zugabe des DMC-Katalysators außerdem zusätzlich geringe Mengen (1 bis 500 ppm) anderer organischer oder anorganischer Säuren zugesetzt werden, wie beispielsweise in WO 99/14258 beschrieben. Die Umsetzung der Komponente A) in Schritt (ii) mit einem oder mehreren Alkylenoxiden B1) unter DMC-Katalyse kann prinzipiell im gleichen Reaktor wie die Herstellung der Komponente A) in den Schritten (i-1) bis (i-3) erfolgen. Die auf die Endproduktmenge (1) berechnete DMC-Katalysatorkonzentration liegt im Bereich von 10 bis 1000 ppm.

DMC-Katalysatoren B2) sind im Prinzip aus dem Stand der Technik bekannt (siehe z.B. US-A 3 404 109, US-A 3829505, US-A 3941849 und US-A 5158922). DMC-Katalysatoren, die z.B. in US-A 5470813, EP-A 700949, EP-A 743093, EP-A 761708, WO 97/40086, WO 98/16310 und WO 00/47649 beschrieben sind, besitzen eine sehr hohe Aktivität in der Polymerisation von Epoxiden und ermöglichen die Herstellung von Polyetherpolyolen bei sehr geringen Katalysatorkonzentrationen (25 ppm oder weniger), so dass eine Abtrennung des Katalysators aus dem fertigen Produkt im Allgemeinen nicht mehr erforderlich ist. Ein typisches Beispiel sind die in EP-A 700949 beschriebenen hochaktiven DMC-Katalysatoren, die neben einer Doppelmetallcyanid-Verbindung (z.B. Zinkhexacyanocobaltat(III)) und einem organischen Komplexliganden (z.B. tert.-Butanol) noch ein Polyetherpolyol mit einer zahlenmittleren Molmasse größer als 500 g/mol enthalten.

Es ist auch möglich, die in EP Anineldenummer 10163170.3 offenbarten alkalischen DMC-Katalysatoren einzusetzen.

Zur Herstellung der Doppelmetallcyanid-Verbindungen geeignete cyanidfreie Metallsalze besitzen bevorzugt die allgemeine Formel (I),

$$M(X)_n \qquad (I)$$

wobei

M ausgewählt ist aus den Metallkationen $Zn^{2+}$, $Fe^{2+}$, $Ni^{2+}$, $Mn^{2+}$, $Co^{2+}$, $Sr^{2+}$, $Sn^{2+}$, $Pb^{2+}$ und, $Cu^{2+}$, bevorzugt ist M $Zn^{2+}$, $Fe^{2+}$, $Co^{2+}$ oder $Ni^{2+}$,
X sind ein oder mehrere (d.h.verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

n ist 1, wenn X = Sulfat, Carbonat oder Oxalat ist und

n ist 2, wenn X = Halogenid, Hydroxid, Cyanat, Thiocyanat, Isocyanat, Isothiocyanat oder Nitrat ist,

oder geeignete cyanidfreie Metallsalze besitzen die allgemeine Formel (II),

$$M_r(X)_3 \qquad (II)$$

wobei

M ausgewählt ist aus den Metallkationen $Fe^{3+}$, $Al^{3+}$ und $Cr^{3+}$,

X sind ein oder mehrere (d.h.verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

r ist 2, wenn X = Sulfat, Carbonat oder Oxalat ist und

r ist 1, wenn X = Halogenid, Hydroxid, Cyanat, Thiocyanat, Isocyanat, Isothiocyanat, Carboxylat oder Nitrat ist,

oder geeignete cyanidfreie Metallsalze besitzen die allgemeine Formel (III),

$$M(X)_s \qquad (III)$$

wobei

M ausgewählt ist aus den Metallkationen $Mo^{4+}$, $V^{4+}$ und $W^{4+}$,

X sind ein oder mehrere (d.h.verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

s ist 2, wenn X = Sulfat, Carbonat oder Oxalat ist und

s ist 4, wenn X = Halogenid, Hydroxid, Cyanat, Thiocyanat, Isocyanat, Isothiocyanat, Carboxylat oder Nitrat ist,

oder geeignete cyanidfreie Metallsalze besitzen die allgemeine Formel (IV),

$$M(X)_t \qquad (IV)$$

wobei

M ausgewählt ist aus den Metallkationen $Mo^{6+}$ und $W^{6+}$,

X sind ein oder mehrere (d.h. verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

t ist 3, wenn X = Sulfat, Carbonat oder Oxalat ist und

t ist 6, wenn X = Halogenid, Hydroxid, Cyanat, Thiocyanat, Isocyanat, Isothiocyanat, Carboxylat oder Nitrat ist,

Beispiele geeigneter cyanidfreier Metallsalze sind Zinkchlorid, Zinkbromid, Zinkjodid, Zinkacetat, Zinkacetylacetonat, Zinkbenzoat, Zinknitrat, Eisen(II)sulfat, Eisen(II)bromid, Eisen(II)chlorid, Cobalt(II)chlorid, Cobalt(II)thiocyanat, Nickel(II)chlorid und Nickel-(II)nitrat. Es können auch Mischungen verschiedener Metallsalze eingesetzt werden.

Zur Herstellung der Doppelmetallcyanid-Verbindungen geeignete Metallcyanidsalze besitzen bevorzugt die allgemeine Formel (V)

$$(Y)_a M'(CN)_b (A)_c \qquad (V)$$

wobei

M' ausgewählt ist aus einem oder mehreren Metallkationen der Gruppe bestehend aus Fe(II), Fe(III), Co(II), Co(III), Cr(II), Cr(III), Mn(II), Mn(III), Ir(III), Ni(II), Rh(III), Ru(II), V(IV) und V(V), bevorzugt ist M' ein oder mehrere Metallkationen der Gruppe bestehend aus Co(II), Co(III), Fe(II), Fe(III), Cr(III), Ir(III) und Ni(II),

Y ausgewählt ist aus einem oder mehreren Metallkationen der Gruppe bestehend aus Alkalimetall (d.h. $Li^+$, $Na^+$, $K^+$, $Rb^+$, $Cs^+$) und Erdalkalimetall (d.h. $Be^{2+}$, $Ca^{2+}$, $Mg^{2+}$, $Sr^{2+}$, $Ba^{2+}$),

A ausgewählt ist aus einem oder mehreren Anionen der Gruppe bestehend aus Halogeniden (d..h. Fluorid, Chlorid, Bromid, Iodid), Hydroxid, Sulfat, Carbonat, Cyanat, Thiocyanat, Isocyanat, Isothiocyanat, Carboxylat, Oxalat oder Nitrat und

a, b und c sind ganzzahlige Zahlen, wobei die Werte für a, b und c so gewählt sind, dass die Elektroneutralität des Metallcyanidsalzes gegeben ist; a ist vorzugsweise 1, 2, 3 oder 4; b ist vorzugsweise 4, 5 oder 6; c besitzt bevorzugt den Wert 0.

Beispiele geeigneter Metallcyanidsalze sind Kaliumhexacyanocobaltat(III), Kaliumhexacyanoferrat(II), Kaliumhexacyanoferrat(III), Calciumhexacyanocobaltat(III) und Lithiumhexacyanocobaltat(III).

Bevorzugte Doppelmetallcyanid-Verbindungen, die in den erfindungsgemäßen DMC-Katalysatoren enthalten sind, sind Verbindungen der allgemeinen Formel (VI)

$$M_x[M'_{x'}(CN)_y]_z \qquad (VI),$$

worin M wie in Formel (I) bis (IV) und

M' wie in Formel (V) definiert ist, und

x, x', y und z sind ganzzahlig und so gewählt, dass die Elektronenneutralität der Doppelmetallcyanidverbindung gegeben ist.

Vorzugsweise ist

$x=3, x'= 1$, $y = 6$ und $z = 2$,

M = Zn(II), Fe(II), Co(II) oder Ni(II) und

M' = Co(III), Fe(III), Cr(III) oder Ir(III).

Beispiele geeigneter Doppelmetallcyanidverbindungen sind Zinkhexacyanocobaltat(III), Zinkhexacyanoiridat(III), Zinkhexacyanoferrat(III) und Cobalt(II)hexacyanocobaltat(III). Weitere Beispiele geeigneter Doppelmetallcyanid-Verbindungen sind z.B. US-A 5158922 (Spalte 8, Zeilen 29 - 66) zu entnehmen. Besonders bevorzugt verwendet wird Zinkhexacyanocobaltat(III).

Die bei der Herstellung der DMC-Katalysatoren zugesetzten organischen Komplexliganden sind beispielsweise in US-A 5158922 (siehe insbesondere Spalte 6, Zeilen 9 bis 65), US-A 3404109, US-A 3829505, US-A 3941849, EP-A 700949, EP-A 761708, JP-A 4145123, US-A 5470813, EP-A 743 093 und WO-A 97/40086) offenbart. Beispielsweise werden als organische Komplexliganden wasserlösliche, organische Verbindungen mit Heteroatomen, wie Sauerstoff, Stickstoff, Phosphor oder Schwefel, die mit der Doppelmetallcyanid-Verbindung Komplexe bilden können, eingesetzt. Bevorzugte organische Komplexliganden sind Alkohole, Aldehyde, Ketone, Ether, Ester, Amide, Harnstoffe, Nitrile, Sulfide und deren Mischungen. Besonders bevorzugte organische Komplexliganden sind aliphatische Ether (wie Dimethoxyethan), wasserlösliche aliphatische Alkohole (wie Ethanol, Isopropanol, n-Butanol, iso-Butanol, sek.-Butanol, tert-Butanol, 2-Methyl-3-buten-2-ol und 2-Methyl-3-butin-2-ol), Verbindungen, die sowohl aliphatische oder cycloaliphatische Ethergruppen wie auch aliphatische Hydroxylgruppen enthalten (wie z.B. Ethylenglykol-mono-tert.-butylether, Diethylenglykol-mono-tert.-butylether, Tripropylenglykol-mono-methylether und 3-Methyl-3-oxetan-methanol). Höchst bevorzugte organische Komplexliganden sind ausgewählt aus einer oder mehrerer Verbindungen der Gruppe bestehend aus Dimethoxyethan, tert-Butanol, 2-Methyl-3-buten-2-ol, 2-Methyl-3-butin-2-ol, Ethylenglykol-mono-tert.-butylether und 3-Methyl-3-oxetan-methanol.

Optional werden bei der Herstellung der erfindungsgemäßen DMC-Katalysatoren eine oder mehrere komplexbildende Komponente(n) aus den Verbindungsklassen der Polyether, Polyester, Polycarbonate, Polyalkylenglykolsorbitanester, Polyalkylenglykolglycidylether, Polyacrylamid, Poly(acrylamid-co-acrylsäure), Polyacrylsäure, Poly(acrylsäure-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly-(acrylsäure-co-styrol), Oxazolinpolymere, Polyalkylenimine, Maleinsäure- und Maleinsäureanhydridcopolymere, Hydroxyethylcellulose und Polyacetale, oder der Glycidylether, Glycoside, Carbonsäureester mehrwertiger Alkohole, Gallensäuren oder deren Salze, Ester oder Amide, Cyclodextrine, Phosphorverbindungen, $\alpha,\beta$-ungesättigten Carbonsäureester oder ionische oberflächen- bzw. grenzflächenaktiven Verbindungen eingesetzt.

Bevorzugt werden bei der Herstellung der erfindungsgemäßen DMC-Katalysatoren im ersten Schritt die wässrigen Lösungen des Metallsalzes (z.B. Zinkchlorid), eingesetzt im stöchiometrischen Überschuss (mindestens 50 Mol-%) bezogen auf Metallcyanidsalz, (also mindestens ein molares Verhältnis von cyanidfreiem Metallsalz zu Metallcyanidsalz von 2,25 zu 1,00) und des Metallcyanidsalzes (z.B. Kaliumhexacyanocobaltat) in Gegenwart des organischen Komplexliganden (z.B. tert.-Butanol) umgesetzt, so dass sich eine Suspension bildet, die die Doppelmetallcyanid-Verbindung (z.B. Zinkhexacyanocobaltat), Wasser, überschüssiges cyanidfreies Metallsalz, und den organischen Komplexliganden enthält. Der organische Komplexligand kann dabei in der wässrigen Lösung des cyanidfreien Metallsalzes und/oder des Metallcyanidsalzes vorhanden sein, oder er wird der nach Ausfällung der Doppelmetallcyanid-Verbindung erhaltenen Suspension unmittelbar zugegeben. Es hat sich als vorteilhaft erwiesen, die wässrigen Lösungen des cyanidfreien Metallsalzes und des Metallcyanidsalzes und den organischen Komplexliganden unter starkem Rühren zu vermischen. Optional wird die im ersten Schritt gebildete Suspension anschließend mit einer weiteren komplexbildenden Komponente behandelt. Die komplexbildende Komponente wird dabei bevorzugt in einer Mischung mit Wasser und organischem Komplexliganden eingesetzt. Ein bevorzugtes Verfahren zur Durchführung des ersten Schrittes (d.h. der Herstellung der Suspension) erfolgt unter Einsatz einer Mischdüse, besonders bevorzugt unter Einsatz eines Strahldispergators wie in WO-A 01/39883 beschrieben.

Im zweiten Schritt erfolgt die Isolierung des Feststoffs (d.h. die Vorstufe des erfindungsgemäßen Katalysators) aus der Suspension durch bekannte Techniken, wie Zentrifugation oder Filtration.

In einer bevorzugten Ausführungsvariante zur Herstellung des Katalysators wird der isolierte Feststoff anschließend in einem dritten Verfahrensschritt mit einer wässrigen Lösung des organischen Komplexliganden gewaschen (z.B. durch Resuspendieren und anschließende erneute Isolierung durch Filtration oder Zentrifugation). Auf diese Weise können zum Beispiel wasserlösliche Nebenprodukte, wie Kaliumchlorid, aus dem erfindungsgemäßen Katalysator entfernt werden. Bevorzugt liegt die Menge des organischen Komplexliganden in der wässrigen Waschlösung zwischen 40 und 80 Gew.-%, bezogen auf die Gesamtlösung. Optional wird im dritten Schritt der wässrigen Waschlösung weitere komplexbildende Komponente, bevorzugt im Bereich zwischen 0,5 und 5 Gew.-%, bezogen auf die Gesamtlösung, zugefügt.

Außerdem ist es vorteilhaft, den isolierten Feststoff mehr als einmal zu waschen. Hierzu kann z.B. der erste Waschvorgang wiederholt werden. Bevorzugt ist es aber, für weitere Waschvorgänge nicht wässrige Lösungen zu verwenden, z.B. eine Mischung aus organischem Komplexliganden und weiterer komplexbildender Komponente.

Der isolierte und gegebenenfalls gewaschene Feststoff wird anschließend, gegebenenfalls nach Pulverisierung, bei Temperaturen von im allgemeinen 20 - 100 °C und bei Drücken von im allgemeinen 0,1 mbar bis Normaldruck (1013 mbar) getrocknet.

Ein bevorzugtes Verfahren zur Isolierung der erfindungsgemäßen DMC-Katalysatoren aus der Suspension durch Filtration, Filterkuchenwäsche und Trocknung wird in WO-A 01/80994 beschrieben.

Der DMC-katalysierte Reaktionsschritt (ii) kann generell nach denselben verfahrenstechnischen Prinzipien durchgeführt werden wie die unter basischer Katalyse erfolgte Herstellung der Komponete A) in den Schritten (i-1) bis (i-3). Auf einige verfahrenstechnische Besonderheiten des DMC-katalysierten Reaktionsschrittes (ii) soll im Folgenden eingegangen werden.

In einer Ausführungsform wird Komponente A) mit DMC-Katalysator versetzt. Nach Aufheizen auf Temperaturen von 60 bis 160 °C, bevorzugt 100 bis 140 °C, ganz besonders bevorzugt 120 bis 140 °C wird der Reaktorinhalt in einer bevorzugten Verfahrensvariante mit Inertgas über einen Zeitraum von bevorzugt 10 bis 60 min. unter Rühren gestrippt. Beim Strippen mit Inertgas werden flüchtige Bestandteile unter Einleiten von Inertgasen in die Flüssigphase bei gleichzeitig angelegtem Vakuum, bei einem absoluten Druck von 5 bis 500 mbar, entfernt. Nach Eindosieren von typischerweise 5 bis 20 Gew.-% Alkylenoxid, bezogen auf die Menge an vorgelegter Komponente A), wird der DMC-Katalysator aktiviert. Die Zugabe eines oder mehrerer Alkylenoxide kann vor, während oder nach dem Aufheizen des Reaktorinhaltes auf Temperaturen von 60 bis 160 °C, bevorzugt 100 bis 140 °C, ganz besonders bevorzugt 120 bis 140 °C geschehen; sie erfolgt bevorzugt nach dem Strippen. Die Aktivierung des Katalysators macht sich durch einen beschleunigten Abfall des Reaktordruckes bemerkbar, wodurch der beginnende Alkylenoxidumsatz angezeigt wird. Dem Reaktionsgemisch kann sodann die gewünschte Menge Alkylenoxid bzw. Alkylenoxidgemisch kontinuierlich zugeführt werden, wobei eine Reaktionstemperatur von 20 bis 200 °C, bevorzugt aber von 50 bis 160 °C gewählt wird. In vielen Fällen erfolgt die Katalysatoraktivierung bereits so schnell, dass die Dosierung einer separaten Menge Alkylenoxid zur Katalysatoraktivierung entfallen kann und direkt mit der kontinuierlichen Dosierung eines Alkylenoxides oder mehrerer Alkylenoxide begonnen werden kann. Auch im DMC-katalysierten Reaktionsschritt kann die Reaktionstemperatur während der Alkylenoxiddosierphase innerhalb der beschriebenen Grenzen variiert werden. Ebenfalls können ein oder mehrere Alkylenoxide dem Reaktor im DMC-katalysierten Reaktionsschritt auf unterschiedliche Weise zugeführt werden: Möglich ist eine Dosierung in die Gasphase oder direkt in die Flüssigphase, z. B. über ein Tauchrohr oder einen in der Nähe des Reaktorbodens in einer gut durchmischten Zone befindlichen Verteilerring. Bei DMC-katalysierten Prozessen ist die Dosierung in die Flüssigphase die bevorzugte Variante.

Nach Ende der Alkylenoxiddosierung kann sich eine Nachreaktionsphase anschließen, in der die Abnahme der Konzentration an unreagiertem Alkylenoxid durch Überwachung des Drucks quantifiziert werden kann. Gegebenenfalls kann das Reaktionsgemisch nach Ende der Nachreaktionsphase von kleinen Mengen an nicht umgesetzten Alkylenoxiden beispielsweise im Vakuum, bei einem abslouten Druck von 1 bis 500 mbar, oder durch Strippen quantitativ entfernt werden. Durch Strippen werden flüchtige Bestandteile, wie beispielsweise (Rest-)Alkylenoxide, unter Einleiten von Inertgasen oder Wasserdampf in die Flüssigphase bei gleichzeitig angelegtem Vakuum (beispielsweise durch Durchleiten von Inertgas bei einem Absolutdruck von 5 bis 500 mbar) entfernt. Das Entfernen flüchtiger Bestandteile, wie beispielsweise nicht umgesetzter Alkylenoxide, entweder im Vakuum oder durch Strippen, erfolgt bei Temperaturen von 20 bis 200 °C, bevorzugt bei 50 bis 160 °C und vorzugsweise unter Rühren. Solche Strippvorgänge können auch in sog. Strippkolonnen durchgeführt werden, in denen dem Produktstrom ein Inertgas- oder Wasserdampfstrom entgegengeleitet wird. Nach Erreichen von Druckkonstanz bzw. nach Entfernen flüchtiger Bestandteile durch Vakuum und/oder Strippen kann das Produkt aus dem Reaktor abgelassen werden.

In einer weiteren Ausführungsform des erfindungsgemäßen Verfahrens wird in Schritt (ii) ein Starterpolyol und DMC-Katalysator B2) im Reaktorsystem vorgelegt und Komponente A) wird kontinuierlich gemeinsam mit einem oder mehreren Alkylenoxiden B1) zugeführt. Als Starterpolyol in Schritt (ii) sind Alkylenoxidadditionsprodukte wie beispielsweise Polyetherpolyole, Polycarbonatpolyole, Polyestercarbonatpolyole, Polyethercarbonatpolyole jeweils beispielsweise mit OH-Zahlen im Bereich von 3 bis 1000 mg KOH/g, vorzugsweise von 3 bis 300 mg KOH/g, eine Teilmenge an Komponente A), und/oder erfindungsgemäßes Endprodukt (1), das vorher separat hergestellt wurde, geeignet. Vorzugsweise wird eine Teilmenge an Komponente A) oder erfindungsgemäßes Endprodukt (1), das vorher separat hergestellt wurde als Starterpolyol in Schritt (ii) eingesetzt. Besonders bevorzugt wird erfindungsgemäßes Endprodukt (1), das vorher separat hergestellt wurde, als Starterpolyol in Schritt (ii) eingesetzt.

Wird die Zusammensetzung der Alkylenoxide nach Ende der Dosierung der Komponente A geändert, lassen sich auch mit dieser Verfahrensweise Polyetherpolyole mit Multiblockstrukturen herstellen. Es ist aber auch möglich, die Komponente A) - Dosierung und die Alkylenoxiddosierung gleichzeitig enden zu lassen. Nach Zudosierung der Reagenzien kann sich eine Nachreaktionsphase anschließen, in der der Verbrauch an Alkylenoxid im Allgemeinen durch Überwachung des Drucks quantifiziert werden kann. Nach Erreichen von Druckkonstanz kann das Produkt, gegebenenfalls wie oben beschrieben nach Anlegen von Vakuum oder durch Strippen zur Entfernung von nicht umgesetzten Alkylenoxiden, abgelassen werden.

Es ist auch möglich in Schritt (ii) die gesamte Menge an Komponente A) und DMC-Katalysator vorzulegen und eine oder mehrere H-funktionelle Starterverbindungen, insbesondere solche mit Äquivalentmolmassen beispielsweise im Bereich von 9,0 bis 350 Da, bevorzugt von 30,0 bis 350 Da, kontinuierlich gemeinsam mit einem oder mehreren Alkylenoxiden B1) zuzuführen.

In einer weiteren Ausführungsform des erfindungsgemäßen Verfahrens wird das Reaktionsprodukt (1) kontinuierlich dem Reaktor entnommen. In dieser Verfahrensweise wird in Schritt (ii) ein Starterpolyol und eine Teilmenge an DMC-Katalysator B2) im Reaktorsystem vorgelegt und Komponente A) wird kontinuierlich gemeinsam mit einem oder mehreren Alkylenoxiden B1) und DMC-Katalysator B2) zugeführt und das Reaktionsprodukt (1) wird dem Reaktor kontinuierlich entnommen. Als Starterpolyol in Schritt (ii) sind Alkylenoxidadditionsprodukte wie beispielsweise Polyetherpolyole, Polycarbonatpolyole, Polyestercarbonatpolyole, Polyethercarbonatpolyole beispielsweise mit OH-Zahlen im Bereich von 3 bis 1000 mg KOH/g, vorzugsweise von 3 bis 300 mg KOH/g, eine Teilmenge an Komponente A), und/oder erfindungsgemäßes Endprodukt (1), das vorher separat hergestellt wurde, geeignet. Vorzugsweise wird eine Teilmenge an Komponente A) oder erfindungsgemäßes Endprodukt (1), das vorher separat hergestellt wurde als Starterpolyol in Schritt (ii) eingesetzt. Besonders bevorzugt wird als Starterpolyol in Schritt (ii) erfindungsgemäßes Endprodukt (1), das vorher separat hergestellt wurde, eingesetzt. Hierbei können sich kontinuierliche Nachreaktionsschritte, beispielsweise in einer Reaktorkaskade oder in einem Rohrreaktor anschließen. Flüchtige Bestandteile können im Vakuum und/oder durch Strippen, wie oben beschrieben, entfernt werden.

Die verschiedenen Verfahrensvarianten bei der Herstellung von Polyethern nach den Alkylenoxidadditionsverfahren unter DMC-Komplexkatalyse sind beispielsweise beschrieben in WO-A 97/29146 und WO-A 98/03571.

Vorzugsweise verbleibt der DMC-Katalysator im Endprodukt, er kann jedoch auch abgetrennt werden, beispielsweise durch Behandlung mit Adsorbentien. Verfahren zur Abtrennung von DMC-Katalysatoren sind beispielsweise beschrieben in US-A 4987271, DE-A 3132258, EP-A 406440, US-A 5391722, US-A 5099075, US-A 4721818, US-A 4877906 und EP-A 385619.

Für das erfindungsgemäße Verfahren können sowohl für den basisch katalysierten Alkylenoxidadditionsschritt (i-1) zum Erhalt der Komponente A1) als auch für den DMC-katalysierten Alkylenoxidadditionsschritt (ii) Alkylenoxide (Epoxide) mit 2 bis 24 Kohlenstoffatomen eingesetzt werden. Bei den Alkylenoxiden mit 2 bis 24 Kohlenstoffatomen handelt es sich beispielsweise um eine oder mehrere Verbindungen ausgewählt aus der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Butadienmonoxid, Isoprenmonoxid, Cyclopentenoxid, Cyclohexenoxid, Cycloheptenoxid, Cyclooctenoxid, Styroloxid, Methylstyroloxid, Pinenoxid, ein- oder mehrfach epoxidierte Fette als Mono-, Di- und Triglyceride, epoxidierte Fettsäuren, $C_1$-$C_{24}$-Ester von epoxidierten Fettsäuren, Epichlorhydrin, Glycidol, und Derivate des Glycidols wie beispielsweise Methylglycidylether, Ethylglycidylether, 2-Ethylhexylglycidylether, Allylglycidylether, Glycidylmethacrylat sowie epoxidfunktionelle Alkyloxysilane wie beispielsweise 3-Glycidyloxypropyltrimethoxysilan, 3-Glycidyloxypropyltriethoxysilan, 3-Glycidyloxypropyltripropoxysilan, 3-Glycidyloxypropyl-methyldimethoxysilan, 3-Glycidyloxypropyl-ethyldiethoxysilan, 3-Glycidyloxypropyltriisopropoxysilan.

Als Alkylenoxide A1.2) für die Herstellung der Polyetherpolyole A1) werden bevorzugt Ethylenoxid und/oder Propylenoxid, bevorzugt mindestens 10 % Ethylenoxid und ganz besonders bevorzugt reines Ethylenoxid eingesetzt.

Als Alkylenoxide B1) in Schritt (ii) werden vorzugsweise Ethylenoxid und/oder Propylenoxid eingesetzt.

Nach dem erfindungsgemäßen Verfahren ist es bevorzugt, dass die Zusammensetzung des Alkylenoxidgemisches beim Wechsel vom basisch katalysierten (i-1) bis (i-3) zum DMC-katalysierten Alkylenoxidadditionsschritt (ii) ebenfalls geändert wird. Werden während des DMC-katalysierten Alkylenoxidadditionschrittes (ii) verschiedene Alkylenoxide verwendet, so können diese wiederum entweder als Mischung oder nacheinander zudosiert werden. Bei letzterer Dosierweise erhalten die unter DMC-Katalyse weiterwachsenden Polyetherketten kompliziertere Blockstrukturen. Zum Erhalt definierter DMC-Blockstrukturen nach dem Verfahren des kontinuierlichen Starter-Komponente-A)-Zudosierungsverfahrens sollte die kontinuierliche Starter-Komponente-A)-Zudosierung gemeinsam mit oder kurz vor Ende der Dosierung des ersten Alkylenoxidblocks beendet werden. Oft werden als Endblock reines Ethylenoxid oder Mischungen aus Propylenoxid und Ethylenoxid mit hohem Ethylenoxidanteil zudosiert, so dass die hergestellten Polyetherpolyole 40 bis 100 % primäre OH-Endgruppen aufweisen.

Weitere nach dem erfindungsgemäßen Verfahren mit Alkylenoxiden unter DMC-Katalyse copolymerisierbare Monomere sind beispielsweise Lactone, Lactide, Säureanhydride, cyclische Carbonate und Kohlendioxid. Ihre Verwendung wird beschrieben in US-A 3538043, US-A 4500704, US-A 5032671, US-A 6646100, EP-A 222453 und WO-A 2008/013731.

Die OH-Zahlen der nach dem DMC-katalysierten Alkylenoxidadditionsschritt (ii) erhaltenen Polyetherpolyole (1) weisen Werte von 3 mg KOH/g bis 150 mg KOH/g, bevorzugt 10 bis 60 mg KOH/g, besonders bevorzugt 20 bis 50 mg KOH/g, auf. Den Endprodukten können ebenfalls gegebenenfalls Alterungsschutzmittel wie z. B. Antioxidantien zugesetzt werden. Die Polyetherpolyole (1) können allein oder gegebenenfalls im Gemisch mit weiteren isocyanatreaktiven Komponenten mit organischen Polyisocyanaten, gegebenenfalls in Gegenwart von Treibmitteln, in Gegenwart von Katalysatoren und gegebenenfalls mit weiteren Zusatzstoffen wie z.B. Zellstabilisatoren zur Reaktion gebracht werden und so als Komponenten von massiven oder geschäumten Polyurethanen, insbesondere Polyurethan-Weichschaum wie beispielsweise Polyurethan-Weichblockschaum und Polyurethan-Weichformschaum, dienen.

Polyurethane, bevorzugt massive oder geschäumte Polyurethane, insbesondere Polyurethan-Weichschäume wie beispielsweise Polyurethan-Weichblockschäume und Polyurethan-Weichformschäume, enthaltend die erfindungsgemäßen Polyetherpolyole (1) sind ebenfalls Gegenstand der Erfindung.

### Beispiele

Bestimmung des Gehaltes an hochmolekularen Verunreinigungen

Die Bestimmung des Gehaltes and hochmolekularen Verunreinigungen erfolgte in Anlehnung an die in US 6013596 beschriebene Methode.

OH-Zahl und Viskosität

Die Bestimmung der OH-Zahlen erfolgte gemäß der Vorschrift der DIN 53240. Die Viskositäten wurden mittels Rotationsviskosimeter (Physica MCR 51, Hersteller: Anton Paar) nach der Vorschrift der DIN 53018 ermittelt.

### Molmassenverteilung

Die Molmassenverteilung wurde mittels Größenausschlusschromatographie (SEC) ermittelt. Verwendet wurde das Gerät Agilent 1100 Series der Fa. Agilent. Angegeben wird die Polydispersität PD für die Molmassenverteilung $M_w/M_n$, wobei $M_w$ für die gewichtsgemittelte Molmasse und $M_n$ für die zahlengemittelte Molmasse stehen. Weitere Angaben:

- Säulenkombination: 1 Vorsäule PSS, 5 $\mu$l, 8x50mm; 2 PSS SVD, 5 $\mu$l, 100 A°, 8x300mm; 2 PSS SVD, 5 $\mu$l, 1000 A°, 8x300mm, PSS ist der Hersteller der Säulen (Polymer Standard Solutions, Mainz)
- Auswertesoftware: WIN GPC der Fa. PSS
- Lösungsmittel: THF (Merck LiChrosolv)
- Flussrate: 1 ml / min
- Detektortyp: RI-Detektor (Brechungsindex), Shodex RI 74
- Verwendete Kalibrationsstandards: Kalibrierstandard der Fa. PSS auf Basis Polystyrol.

**Eingesetzte Rohstoffe**

**Katalysator für die Alkylenoxidaddition (DMC-Katalysator):**

Doppelmetallcyanid-Katalysator, enthaltend Zinkhexacyanocobaltat, tert.-Butanol und Polypropylenglykol mit einer zahlenmittleren Molmasse von 1000 g/mol; beschrieben in WO-A 01/80994, Beispiel 6.

**Herstellung des polymeren Alkoxylates I (basischer Katalysator zur Herstellung der Verbindungen A1))**

In einen 10 l Laborautoklaven wurden unter Stickstoffatmosphäre 3677,2 g Glycerin und 13,33 g einer 45 Gew.-%igen Lösung von KOH in Wasser gegeben. Der Autoklav wurde verschlossen, die Rührerdrehzahl auf 450 U/min. eingestellt und man heizte auf 110 °C auf. Der absolute Druck wurde auf 100 mbar abgesenkt und 2313,7 g Propylenoxid wurden über einen Zeitraum von 4,6 h in den Autoklaven dosiert. Nach einer Nachreaktionszeit von 180 min. bei 110 °C wurde der absolute Druck langsam wieder auf 100 mbar abgesenkt und der Ansatz schließlich im Vakuum bei einem absoluten Druck von 18 mbar von Wasser befreit, bis bei einer Temperatur von 110°C ein absoluter Druck von 10 mbar erreicht war. Die Alkalizahl des polymeren Alkoxylates I beträgt 1,0 mg KOH/g, sein KOH-Gehalt entsprechend 0,1 %. Seine OH-Zahl beträgt 1121 mg KOH/g. Der Alkoxylatgehalt beträgt entsprechend 0,09 %.

**Herstellung des polymeren Alkoxylates II (basischer Katalysator zur Herstellung der Verbindungen A1))**

In einen 10 l Laborautoklaven wurden unter Stickstoffatmosphäre 1278,5 g Trimethylolpropan und 21,7 g einer 45 Gew.-%igen Lösung von KOH in Wasser gegeben. Der Autoklav wurde verschlossen, die Rührerdrehzahl auf 450 U/min. eingestellt und man heizte auf 107 °C auf. Der absolute Druck wurde auf 100 mbar abgesenkt und 653,4 g Propylenoxid wurden über einen Zeitraum von 3 h in den Autoklaven dosiert. Nach einer Nachreaktionszeit von 30 min. bei 107 °C wurde der Ansatz 30 min. bei einem absoluten Druck von 10 mbar ausgeheizt. Nach Abkühlen auf 25 °C wurden unter Stickstoffatmosphäre 45,1 g einer 45 Gew.-%igen Lösung von KOH in Wasser zugegeben. Man erwärmte auf 107 °C und entfernte das Wasser im Vakuum, bis ein absoluter Druck von 10 mbar erreicht war. Sodann wurden 4063,6 g Propylenoxid über einen Zeitraum von 8,5 h bei 107 °C eindosiert, nach einer Nachreaktionszeit von 120 min. wurde für 30 min. im Vakuum bei einem absoluten Druck von 1 mbar ausgeheizt. Nach Abkühlen auf 25 °C wurden unter Stickstoffatmosphäre 539,4 g einer 45 Gew.-%igen Lösung von KOH in Wasser zugegeben. Man erwärmte auf 107 °C und entfernte das Wasser im Vakuum, bis ein absoluter Druck von 10 mbar erreicht war. Die Alkalizahl des polymeren Alkoxylates II beträgt 44,1 mg KOH/g, sein KOH-Gehalt entsprechend 4,41 %. Die OH-Zahl beträgt 260 mg KOH/g. Der Alkoxylatgehalt beträgt entsprechend 17 %.

**IRGANOX® 1076**

Octadecyl-3-(3,5-di-tert.butyl-4-hydroxyphenyl)propionat (BASF SE)

**Beispiel 1**

In einen 10 l Laborautoklaven wurden unter Stickstoffatmosphäre 325,5 g Sorbit und 3,075 g einer 44,82 %igen Lösung von KOH in Wasser gegeben. Der Autoklav wurde verschlossen und sein Inhalt bei 110 °C über einen Zeitraum von 3 h und bei einer Rührerdrehzahl von 450 U/min bei einem absoluten Druck von 100 bis 120 mbar durch Einleiten von 50 ml Stickstoff pro Minute in die Flüssigphase über einen unter dem Flüssigkeitsspiegel liegenden mit 5 Bohrungen versehenen Verteilerring gestrippt Man erwärmte unter Rühren (450 U/min) auf 150 °C und dosierte über einen Zeitraum von 3,22 h 1135,1 g Propylenoxid so in den Autoklaven, dass ein konstanter absoluter Druck von 5 bar erreicht wurde. Nach einer Nachreaktionszeit von 2,45 h wurde der Reaktordruck mit Stickstoff auf einen absoluten Druck von 2,7 bar eingestellt und 4540,2 g Ethylenoxid über einen Zeitraum von 9,07 h eindosiert. Während dieser Dosierzeit wurde die Dosierung bei Erreichen von 5 bar absoluten Reaktordruck zweimal unterbrochen, man ließ jeweils abreagieren, senkte den Druck durch Ablassen des Stickstoffs auf 2,5 bar (absolut) und nahm sodann die Alkylenoxiddosierung wieder auf. Nach Ende der Ethylenoxiddosierung schloss sich eine Nachreaktionszeit von 1,5 h Dauer an. Nach einer Ausheizzeit von 30 min bei einem Druck (absolut) von 10 mbar wurde auf 25 °C abgekühlt. Die auf KOH berechnete Katalysatorkonzentration betrug 230 ppm. 2006,2 g des alkalischen Produktes (A1-1) wurden bei 80 °C mit 6,475 g 11,82 %iger Schwefelsäure versetzt und 0,5 h bei 80 °C verrührt. Nach Zugabe von 1,028 g IRGANOX® 1076 wurde bei 110 °C 3 h bei einem absoluten Druck von 1 mbar entwässert. Man erhielt ein klares Produkt (A-1) mit einer Viskosität von 730 mPas bei 25 °C.

558,3 g des Polyetherpolyoles A-1) wurden unter Stickstoffatmosphäre in einen 10 l Laborautoklaven überführt. Nach Zugabe von 0,051 g DMC-Katalysator wurde der Inhalt des Autoklaven 30 min. bei 130 °C und Rühren (Gitterrührer) mit 450 U/min. im Vakuum bei einem absoluten Druck von 100 bis 120 mbar unter Einleiten von 50 ml Stickstoff pro Minute über einen unter dem Flüssigkeitsspiegel liegenden Verteilerring gestrippt. Über diesen Verteilerring wurden sodann ebenfalls bei 130 °C und Rühren mit 450 U/min. 441,7 g Propylenoxid über einen Zeitraum von 3,05 h eindosiert. Der Start der Propylenoxiddosierung erfolgte bei einem Druck (absolut) von 0,05 bar, der während der Dosierphase erreichte absolute Maximaldruck betrug 0,8 bar. Nach einer Nachreaktionszeit von 0,35 h wurde 0,5 h bei 130 °C bei einem absoluten Druck von 1 mbar ausgeheizt, danach auf 80 °C abgekühlt und 0,535 g IRGANOX® 1076 zugegeben. Die OH-Zahl des Polyetherpolyols 1 betrug 57,2 mg KOH/g und die Viskosität bei 25 °C 1010 mPas. Über Größenausschlusschromatographie (Polystyrolstandards) wurde eine Polydispersität (Mw / Mn) von 1,10 ermittelt. Die Gehalte an hochmolekularen Verunreinigungen sind in Tabelle 1 gelistet.

**Beispiel 2 (Vergleich)**

Die Herstellung des alkalischen Vorproduktes erfolgte in ähnlicher Weise wie in Beispiel 1, lediglich die Katalysatorkonzentration (KOH) wurde von 230 ppm auf 1960 ppm angehoben. 2123 g des alkalischen Vorproduktes (A1-2) wurden bei 80 °C mit 201,5 g Wasser und 31,52 g 11,95 %iger Schwefelsäure versetzt. Nach 0,5 h Rühren bei 80 °C wurden 0,849 g IRGANOX® 1076 zugegeben, das Wasser abdestilliert und 3 h bei einem absoluten Druck von 1 mbar bei 110 °C ausgeheizt. Nach Filtration über ein Tiefenfilter (T 750) bei 80 °C erhielt man ein klares Zwischenprodukt mit einer Viskosität von 720 mPas bei 25 °C.

561,0 g des Zwischenproduktes wurden unter Stickstoffatmosphäre in einen 10 l Laborautoklaven überführt. Nach Zugabe von 0,054 g DMC-Katalysator wurde der Inhalt des Autoklaven 30 min. bei 130 °C und Rühren (Gitterrührer) mit 450 U/min. bei einem absoluten Druck von 100 bis 120 mbar unter Einleiten von 50 ml Stickstoff pro Minute über einen unter dem Flüssigkeitsspiegel liegenden Verteilerring gestrippt. Über diesen Verteilerring wurden sodann ebenfalls bei 130 °C und Rühren mit 450 U/min. 440,0 g Propylenoxid über einen Zeitraum von 2,93 h eindosiert. Der Start der Propylenoxiddosierung erfolgte bei einem absoluten Druck von 0,05 bar, der während der Dosierphase erreichte absolute Maximaldruck betrug 0,75 bar. Nach einer Nachreaktionszeit von 0,33 h wurde 0,5 h bei 130 °C bei einem Druck (absolut) von 10 mbar ausgeheizt, danach auf 80 °C abgekühlt und 0,545 g IRGANOX® 1076 zugegeben. Die OH-Zahl des Polyetherpolyols 2 betrug 58,9 mg KOH/g und die Viskosität bei 25 °C 1010 mPas. Über Größenausschlusschromatographie (Polystyrolstandards) wurde eine Polydispersität (Mw/Mn) von 1,18 ermittelt. Die Gehalte an hochmolekularen Verunreinigungen sind in Tabelle 1 gelistet.

**Tabelle 1:**

| | Beispiel 1 | Beispiel 2 (Vergleich) |
|---|---|---|
| **Mol Schwefelsäure / mol KOH zur Neutralisation des alkalischen Vorproduktes (A1)** | 0,95 | 0,52 |
| **OH-Zahl [mg KOH / g]** | 57,2 | 58,9 |
| **Viskosität bei 25 °C [mPas]** | 1010 | 1010 |
| **Mw/Mn** | 1,10 | 1,18 |
| **Gehalte an hochmolekularen Verunreinigungen [ppm]:** | | |
| 40.000 - 100.000 Da | 801 | 3790 |
| 100.000 - 200.000 Da | 0 | 216 |
| > 200.000 Da | 0 | 0 |

**Beispiel 3**

In einen 10 l Laborautoklaven wurden unter Stickstoffatmosphäre 820,7 g Glycerin und 1,471 g einer 44,82 %igen Lösung von KOH in Wasser gegeben. Der Autoklav wurde verschlossen und sein Inhalt bei 110 °C über einen Zeitraum von 3 h und bei einer Rührerdrehzahl von 450 U/min (Gitterrührer) bei einem absoluten Druck von 100 bis 120 mbar unter Einleiten von 50 ml Stickstoff pro Minute über einen unter dem Flüssigkeitsspiegel liegenden Verteilerring gestrippt. Man erwärmte unter Rühren (450 U/min) auf 150 °C und beaufschlagte den Autoklaven bis zu einem absoluten Druck von 2,5 bar mit Stickstoff. Sodann dosierte man über einen Zeitraum von 10,53 h ein Gemisch aus 1289,2 g Propylenoxid und 3884,5 g Ethylenoxid so in den Autoklaven, dass maximal ein absoluter Druck von 5 bar erreicht wurde. Nach Ende der Alkylenoxiddosierung schloss sich eine Nachreaktionszeit von 3,25 h Dauer an. Nach einer Ausheizzeit von 30 min bei einem absoluten Druck von 10 mbar wurde auf 25 °C abgekühlt. Die auf KOH berechnete Katalysatorkonzentration betrug 110 ppm. 2417,1 g des Reaktionsproduktes A1-3 wurden unter Stickstoffatmosphäre bei 80 °C mit 3,547 g 11,80 %iger Schwefelsäure versetzt und 1 h bei 80 °C verrührt. Nach Zugabe von 1,210 g Irganox® 1076 wurde das Produkt 1 h bei einem absoluten Druck von 18 mbar (Wasserstrahlvakuum) und anschließend bei 110 °C und einem Druck (absolut) von 1 mbar 3 h entwässert. Man erhielt ein klares Produkt (A-3) mit einer Viskosität von 231 mPas bei 25 °C.

896,1 g des Produktes A-3) wurden unter Stickstoffatmosphäre in einen 10 l Laborautoklaven überführt. Nach Zugabe von 0,1054 g Phosphorsäure wurde bei Raumtemperatur 20 min. lang gerührt. Danach wurden 0,185 g DMC-Katalysator zugegeben und der Inhalt des Autoklaven 30 min. bei 130 °C und Rühren mit 450 U/min. im Vakuum bei einem absoluten Druck von 100 bis 120 mbar unter Einleiten von 50 ml Stickstoff pro Minute über einen unter dem Flüssigkeitsspiegel liegenden Verteilerring gestrippt. Über diesen Verteilerring wurden sodann ebenfalls bei 130 °C und Rühren mit 450 U/min. ein Gemisch aus 1275,3 g Propylenoxid und 3828,1 g Ethylenoxid über einen Zeitraum von 6,02 h eindosiert. Der Start der Propylenoxiddosierung erfolgte bei einem absoluten Druck von 2,5 bar, der während der Dosierphase erreichte absolute Maximaldruck betrug 3,94 bar. Nach einer Nachreaktionszeit von 0,4 h wurde 0,55 h bei 130 °C bei einem Druck (absolut) von 10 mbar ausgeheizt, danach auf 80 °C abgekühlt und 3,008 g IRGANOX® 1076 zugegeben. Die OH-Zahl des Polyetherpolyols 3 betrug 36,5 mg KOH/g und die Viskosität bei 25 °C 1380 mPas. Über Größenausschlusschromatographie (Polystyrolstandards) wurde eine Polydispersität (Mw / Mn) von 1,30 ermittelt.

**Beispiel 4 (Vergleich)**

Die Herstellung des alkalischen Vorproduktes A1-4 erfolgte wie in Beispiel 3. 2148,2 g des alkalischen Vorproduktes A1-4 wurden bei 80 °C mit 2,5888 g 10,35 %iger Salpetersäure versetzt. Nach 1 h Rühren bei 80 °C wurden 1,081 g IRGANOX® 1076 zugegeben und 3 h bei einem Druck (absolut) von 1 mbar bei 110 °C ausgeheizt. Man erhielt man ein klares Produkt A-4 mit einer Viskosität von 229 mPas bei 25 °C.

500 g des Produktes A-4 wurden unter Stickstoffatmosphäre in einen 10 l Laborautoklaven überführt. Nach Zugabe von 0,093 g Phosphorsäure wurde bei 25 °C 20 min. lang gerührt. Danach wurden 0,120 g DMC-Katalysator zugegeben und der Inhalt des Autoklaven 30 min. bei 130 °C und Rühren mit 450 U/min. bei einem absoluten Druck von 100 bis 120 mbar unter Einleiten von 50 ml Stickstoff pro Minute über einen unter dem Flüssigkeitsspiegel liegenden Verteilerring gestrippt. Über diesen Verteilerring wurden sodann ebenfalls bei 130 °C und Rühren mit 450 U/min. 1198 g Propylenoxid über einen Zeitraum von 4,33 h eindosiert: Der Start der Propylenoxiddosierung erfolgte bei einem absoluten Druck von 0,05 bar, nach anfänglicher Aktivierung des DMC-Katalysators, erkennbar durch einen beschleunigten Druckabfall nach Abstoppen der Propylenoxiddosierung, stieg der absolute Druck während der Dosierphase kontinuierlich an, so dass die Dosierung 2 mal unterbrochen werden musste und schließlich nach den obengenannten 1198 g dosierten Propylenoxids (70 % der ursprünglich beabsichtigten Propylenoxidmenge) abgebrochen wurde.

**Beispiel 5**

In einen 10 l Laborautoklaven wurden unter Stickstoffatmosphäre 466,5 g des polymeren Alkoxylates I und 560,4 g Glycerin gegeben. Der Autoklav wurde verschlossen und sein Inhalt unter Rühren (Gitterrührer) auf 150 °C bei einer Rührerdrehzahl von 450 U/min erwärmt. Nach Erreichen dieser Temperatur beaufschlagte man den Autoklaven mit Stickstoff bis zu einem absoluten Druck von 2,6 bar. Sodann dosierte man über einen Zeitraum von insgesamt 11,2 h 5178,3 g Ethylenoxid so in den Autoklaven, dass maximal ein absoluter Druck von 5 bar erreicht wurde. Aufgrund der durch den steigenden Füllstand bedingten Kompression des Gasraums musste die Ethylenoxiddosierung 4 mal unterbrochen werden, man ließ jeweils auf ein konstantes Druckniveau abreagieren und stellte vor Dosierung der nächsten Portion den absoluten Druck erneut auf 2,6 bar ein. Nach Ende der Ethylenoxiddosierung schloss sich eine Nachreaktionszeit von 1,42 h Dauer an. Nach einer Ausheizzeit von 30 min im Vakuum wurde auf 25 °C abgekühlt. Die auf KOH berechnete Katalysatorkonzentration betrug 100 ppm. 2938,8 g des Reaktionsproduktes A1-5 wurden unter Stickstoffatmosphäre bei 80 °C mit 3,910 g 11,82 %iger Schwefelsäure versetzt und 1 h bei 80 °C verrührt. Nach Zugabe von 1,472 g Irganox® 1076 wurde das Produkt 1 h bei einem absoluten Druck von 18 mbar (Wasserstrahlvakuum) entwässert und anschließend bei 110 °C und einem absoluten Druck von 1 mbar 3 h ausgeheizt. Man erhielt ein klares Produkt A-5 mit einer Viskosität von 235 mPas bei 25 °C.

260,4 g des Produktes A-5 wurden unter Stickstoffatmosphäre in einen 2 l Laborautoklaven überführt. Nach Zugabe von 0,061 g DMC-Katalysator wurde der Inhalt des Autoklaven 30 min. bei 130 °C und Rühren (mehrstufiger Propellerrührer) mit 450 U/min bei einem absoluten Druck von 100 bis 120 mbar unter Einleiten von 50 ml Stickstoff pro Minute über einen unter dem Flüssigkeitsspiegel liegenden Verteilerring gestrippt. Über diesen Verteilerring wurden sodann ebenfalls bei 130 °C und Rühren mit 450 U/min. 939,6 g Propylenoxid über einen Zeitraum von 4,65 h eindosiert. Der Start der Propylenoxiddosierung erfolgte bei einem absoluten Druck von 0,05 bar, der während der Dosierphase erreichte absolute Maximaldruck betrug 1,2 bar. Nach einer Nachreaktionszeit von 0,33 h wurde 0,5 h bei 130 °C bei einem Druck (absolut) von 10 mbar ausgeheizt, danach auf 80 °C abgekühlt und 0,628 g IRGANOX® 1076 zugegeben.

Die OH-Zahl des Polyetherpolyols 5 betrug 53,4 mg KOH/g und die Viskosität bei 25 °C 602 mPas. Über Größenausschlusschromatographie (Polystyrolstandards) wurde eine Polydispersität (Mw/Mn) von 1,03 ermittelt. Die Gehalte an hochmolekularen Verunreinigungen sind in Tabelle 2 gelistet.

**Beispiel 6 (Vergleich)**

Die Herstellung des alkalischen Vorproduktes A1-6 erfolgte wie in Beispiel 5. 2869,1 g des alkalischen Produktes A1-6 wurden bei 80 °C mit 2,5376 g 20,35 %iger Perchlorsäure versetzt. Nach 1 h Rühren bei 80 °C wurde das Produkt 1 h bei einem Druck (absolut) von 18 mbar (Wasserstrahlvakuum) entwässert und anschließend, nach Zugabe von 1,455 g IRGANOX® 1076, bei 110 °C und bei einen Druck (absolut) von 1 mbar über einen Zeitraum von 3 h ausgeheizt. Man erhielt man ein klares Produkt A-6 mit einer Viskosität von 236 mPas bei 25 °C.

259,6 g des Produktes A-6 wurden unter Stickstoffatmosphäre in einen 2 l Laborautoklaven überführt. Nach Zugabe von 0,060 g DMC-Katalysator wurde der Inhalt des Autoklaven 30 min. bei 130 °C und Rühren (mehrstufiger Propellerrührer) mit 450 U/min bei einem absoluten Druck von 100 bis 120 mbar unter Einleiten von 50 ml Stickstoff/min. über einen unter dem Flüssigkeitsspiegel liegenden Verteilerring gestrippt. Über diesen Verteilerring wurden sodann ebenfalls bei 130 °C und Rühren mit 450 U/min. 940,4 g Propylenoxid über einen Zeitraum von 4,65 h eindosiert. Der Start der Propylenoxiddosierung erfolgte bei einem absoluten Druck von 0,05 bar, der während der Dosierphase erreichte absolute Maximaldruck betrug 1,3 bar. Nach einer Nachreaktionszeit von 0,97 h wurde 0,5 h bei 130 °C bei einem Druck (absolut) von 10 mbar ausgeheizt, danach auf 80 °C abgekühlt und 0,657 g IRGANOX® 1076 zugegeben. Die OH-Zahl des Polyetherpolyols 6 betrug 53,6 mg KOH/g und die Viskosität bei 25 °C 613 mPas. Über Größenausschlusschromatographie (Polystyrolstandards) wurde eine Polydispersität (Mw/Mn) von 1,05 ermittelt. Die Gehalte an hochmolekularen Verunreinigungen sind in Tabelle 2 gelistet.

**Tabelle 2:**

| | Beispiel 5 | Beispiel 6 (Vergleich) |
|---|---|---|
| **Neutralisationssäure (Mol Säure / mol KOH zur Neutralisation des alkalischen Vorproduktes A1)** | Schwefelsäure (0,9) | Perchlorsäure (1,0) |
| **OH-Zahl [mg KOH / g]** | 53,4 | 53,6 |
| **Viskosität bei 25 °C [mPas]** | 602 | 613 |
| **Mw / Mn** | 1,03 | 1,05 |
| **Gehalte an hochmolekularen Verunreinigungen [ppm]:** | | |
| 40.000 - 100.000 Da | 656 | 945 |
| 100.000 - 200.000 Da | 0 | 260 |
| > 200.000 Da | 0 | 0 |

**Beispiel 7**

In einen 10 l Laborautoklaven wurden unter Stickstoffatmosphäre 13,56 g des polymeren Alkoxylates II und 1215,2 g Trimethylolpropan gegeben. Der Autoklav wurde verschlossen und sein Inhalt durch Aufheizen auf 80°C aufgeschmolzen. Restsauerstoff wurde nach Schließen des Befüllstutzens bei 25 °C durch dreimaliges Aufdrücken von Stickstoff bis zu einem absoluten Durck von 3 bar und anschließendes Ablassen des Überdrucks auf Atmosphärendruck entfernt. Anschließend wurde unter Rühren (Gitterrührer) auf 150 °C bei einer Rührerdrehzahl von 450 U/min erwärmt. Nach Erreichen dieser Temperatur beaufschlagte man den Autoklaven mit Stickstoff bis zu einem absoluten Druck von 2,4 bar. Sodann dosierte man über einen Zeitraum von insgesamt 8,41 h 4771,5 g Ethylenoxid so in den Autoklaven, dass maximal ein absoluter Druck von 4,8 bar erreicht wurde. Aufgrund der durch den steigenden Füllstand bedingten Kompression des Gasraums musste die Ethylenoxiddosierung 3 mal unterbrochen werden, man ließ jeweils auf ein konstantes Druckniveau abreagieren und stellte vor Dosierung der nächsten Portion den absoluten Druck erneut auf 2,4 bar ein. Nach Ende der Ethylenoxiddosierung schloss sich eine Nachreaktionszeit von 50 min. Dauer an. Nach einer Ausheizzeit von 30 min bei einem absoluten Druck von 10 mbar wurde auf 25 °C abgekühlt. Die auf KOH berechnete Katalysatorkonzentration betrug 100 ppm. 2783,0 g des Reaktionsproduktes A1-7 wurden unter Stickstoffatmosphäre bei 80 °C mit 3,690 g 11,887 %iger Schwefelsäure versetzt und 1 h bei 80 °C verrührt. Danach wurde das Produkt 1 h bei einem Druck (absolut) von 18 mbar (Wasserstrahlvakuum) entwässert und anschließend bei 110 °C und einem Druck (absolut) von 1 mbar 3 h ausgeheizt. Nach Zugabe von 1,401 g IRGANOX® 1076 erhielt man ein klares Produkt A-7 mit einer Viskosität von 269 mPas bei 25 °C.

100 g des Produktes A-7 wurden unter Stickstoffatmosphäre in einen 1 l Edelstahldruckreaktor überführt. Nach Zugabe von 0,014 g DMC-Katalysator wurde auf 130 °C aufgeheizt und 30 min. bei einem absolutem Druck von 0,1 bar unter Rühren und Durchleiten von 50 ml Stickstoff pro Minute gestrippt. Anschließend wurde bei einem absoluten Reaktordruck von 0,1 bar mit der Dosierung von 364 g Propylenoxid begonnen, wobei der DMC-Katalysator direkt bei Dosierbeginn aktiv war. Die Dosierzeit betrug 30 min. während der der absolute Reaktordruck auf 2,8 bar anstieg. Nach einer Nachreaktionszeit von 30 min. bei 130 °C wurden leichtflüchtige Anteile bei 90 °C über einen Zeitraum von 30 min. bei einem absoluten Druck von 10 mbar abdestilliert und das Reaktionsgemisch anschließend auf 25 °C abgekühlt.

Die OH-Zahl des Polyetherpolyols 7 lag bei 52,4 mg KOH/g bei einer Viskosität (25 °C) von 701 mPas.

## Beispiel 8 (Vergleich)

Die Herstellung des alkalischen Vorproduktes A1-8 erfolgte wie in Beispiel 7. 987,1 g des alkalischen Vorproduktes A1-8 wurden bei 80 °C mit 0,2055 g 85 %iger Phosphorsäure versetzt. Danach wurde das Produkt 1 h bei einem absoluten Druck von 18 mbar (Wasserstrahlvakuum) entwässert und anschließend bei 110 °C und und einem absoluten Druck von 1 mbar 3 h ausgeheizt. Nach Zugabe von 0,495 g IRGANOX® 1076 erhielt man ein klares Produkt A-8 mit einer Viskosität von 268 mPas bei 25 °C.

100 g des Produktes A-8 wurden unter Stickstoffatmosphäre in einen 1 l Edelstahldruckreaktor überführt. Nach Zugabe von 0,014 g DMC-Katalysator wurde auf 130 °C aufgeheizt und 30 min. bei einem absolutem Druck von 0,1 bar unter Rühren und Durchleiten von 50 ml Stickstoff pro Minute gestrippt. Anschließend wurde bei einem absoluten Reaktordruck von 0,1 bar mit der Dosierung von Propylenoxid begonnen. Insgesamt wurden 75 g Propylenoxid bei 130 °C innerhalb von 6 min zudosiert, ohne dass dabei eine Katalysatoraktivierung zu beobachten war. Der absolute Druck im Reaktor stieg während der Propylenoxid-Dosierung auf 5,8 bar an. Nach dem Stoppen der PropylenoxidDosierung trat innerhalb von 30 min keine Aktivierung des Katalysators ein.

## Beispiel 9

In einen 10 l Laborautoklaven wurden unter Stickstoffatmosphäre 1049,0 g Propylenglykol und 2,748 g einer 44,63 %igen Lösung von KOH in Wasser gegeben. Restsauerstoff wurde nach Schließen des Befüllstutzens bei 25 °C durch viermaliges Aufdrücken von Stickstoff bis zu einem absoluten Durck von 3 bar und anschließendes Ablassen des Überdrucks auf Atmosphärendruck entfernt. Man erwärmte unter Rühren (mehrstufiger Propellerrührer mit 450 U/min) auf 150 °C und dosierte über einen Zeitraum von 14 h 3963,0 g Propylenoxid in den Autoklaven. Nach Ende der Alkylenoxiddosierung schloss sich eine Nachreaktionszeit von 6 h Dauer an. Nach einer Ausheizzeit von 30 min bei 150 °C und bei einem absolutem Druck von 10 mbar wurde auf 80°C abgekühlt. Die auf KOH berechnete Katalysatorkonzentration betrug 245 ppm. Das Reaktionsprodukt A1-9 wurde unter Stickstoffatmosphäre bei 80 °C mit 17,862 g 12,01 %iger Schwefelsäure versetzt und 1 h bei 80 °C verrührt. Nach Zugabe von 3,015 g Irganox® 1076 wurde das Produkt 1 h bei einem absolutem Druck von 18 mbar (Wasserstrahlvakuum) und anschließend bei 110 °C und einem absolutem Druck von 1 mbar 3 h entwässert. Das erhaltene Produkt A-9 wies eine Viskosität von 57 mPas bei 25 °C und eine OH-Zahl von 309 mg KOH/g auf.

217,9 g des Produktes A-9 wurden unter Stickstoffatmosphäre in einen 2 l Laborautoklaven überführt. Nach Zugabe von 0,041 g DMC-Katalysator wurde der Inhalt des Autoklaven 30 min. bei 130 °C und Rühren (Propellerrührer) mit 450 U/min bei einem absoluten Druck von 100 bis 120 mbar unter Einleiten von 50 ml Stickstoff pro Minute über einen unter dem Flüssigkeitsspiegel liegenden Verteilerring gestrippt. Über diesen Verteilerring wurden sodann ebenfalls bei 130 °C und Rühren mit 450 U/min. 41,1 g Propylenoxid über einen Zeitraum von 0,42 h eindosiert, man begann die Dosierung bei einem absoluten Druck von 0,05 bar. Nach einer Nachreaktionszeit von 30 min. wurde der Reaktor mit Stickstoff bis einem absoluten Druck von 2,5 bar beaufschlagt. Sodann wurden 342,9 g Ethylenoxid über einen Zeitraum von 1,18 h eindosiert. Hierbei wurde so verfahren, dass maximal ein absoluter Druck von 5 bar erreicht wurde. Nach einer Nachreaktionszeit von 0,25 h wurden weitere 418,3 g Propylenoxid über einen Zeitraum von 3,85 h eindosiert. Es schloss sich einen Nachreaktionszeit von 0,95 h an, das Produkt wurde daraufhin 0,5 h bei 130 °C im Vakuum bei einem absoluten Druck von 10 mbar ausgeheizt, danach auf 80 °C abgekühlt und 0,616 g IRGANOX® 1076 wurden zugegeben. Die OH-Zahl des Polyetherpolyols 9 betrug 66,1 mg KOH/g. Über Größenausschlusschromatographie (Polystyrolstandards) wurde eine Polydispersität (Mw/Mn) von 1,22 ermittelt.

## Beispiel 10

In einen 2 l Laborautoklaven wurden unter Stickstoffatmosphäre 244,3 g Propylenglykol und 0,595 g Imidazol gegeben. Restsauerstoff wurde nach Schließen des Befüllstutzens bei 25 °C durch viermaliges Aufdrücken von Stickstoff bis zu einem absoluten Durck von 3 bar und anschließendes Ablassen des Überdrucks auf Atmosphärendruck entfernt.

Man erwärmte unter Rühren (Ankerrührer mit 800 U/min) auf 105 °C und dosierte über einen Zeitraum von 6 h 901,3 g Ethylenoxid in den Autoklaven. Nach Ende der Ethylenoxiddosierung schloss sich eine Nachreaktionszeit von 0,72 h Dauer an. Nach einer Ausheizzeit von 30 min bei 105 °C bei einem absoluten Druck von 10 mbar wurde auf 80°C abgekühlt. Die Katalysatorkonzentration betrug 519 ppm. Das Reaktionsprodukt A1-10 wurde unter Stickstoffatmosphäre bei 80 °C mit 7,153 g 12,01 %iger Schwefelsäure versetzt und 0,5 h bei 80 °C verrührt. Nach Zugabe von 0,625 g Irganox® 1076 wurde das Produkt 1 h bei einem absoluten Druck von 18 mbar (Wasserstrahlvakuum) und anschließend bei 110°C und bei einem absoluten Druck von 1 mbar 3 h entwässert. Das erhaltene Produkt A-10 wies eine Viskosität von 68 mPas bei 25 °C und eine OH-Zahl von 333 mg KOH/g auf.

101,1 g des Produktes A-10 wurden unter Stickstoffatmosphäre in einen 2 l Laborautoklaven überführt. Nach Zugabe von 0,243 g DMC-Katalysator wurde der Inhalt des Autoklaven 30 min. bei 130 °C und Rühren (Propellerrührer) mit 450 U/min bei einem absoluten Druck von 100 bis 120 mbar unter Einleiten von 50 ml Stickstoff pro Minute über einen unter dem Flüssigkeitsspiegel liegenden Verteilerring gestrippt. Über diesen Verteilerring wurden sodann ebenfalls bei 130 °C und Rühren mit 800 U/min. 1099,8 g Propylenoxid über einen Zeitraum von 7,22 h eindosiert, man begann die Dosierung bei einem absoluten Druck von 0,05 bar. Nach einer Nachreaktionszeit von 30 min. wurde das Produkt 30 min. bei 130 °C bei einem absolutem Druck von 10 mbar ausgeheizt, danach auf 80 °C abgekühlt und 0,631 g IRGANOX® 1076 wurden zugegeben. Die OH-Zahl des Polyetherpolyols 10 betrug 29.2 mg KOH/g. Über Größenausschlusschromatographie (Polystyrolstandards) wurde eine Polydispersität (Mw/Mn) von 1,19 ermittelt.

**Herstellung von Polyurethan-Weichblockschaumstoffen mit Polyetherpolyolen hergestellt nach Beispiel 1 (erfinderisch) und Beispiel 2 (Vergleich)**

Weitere eingesetzte Rohstoffe:

Arcol® 1108:   Polyetherpolyol mit einer OH-Zahl von 48 mg KOH/g, hergestellt über einen vollkontinuierlichen DMC-katalysierten Alkylenoxidadditionsprozess, wobei an ein Gemisch an Starterverbindungen (Glycerin und Propylenglykol im Gewichtsverhältnis 83,5 / 16,5) ein Gemisch aus Propylenoxid und Ethylenoxid im Gewichtsverhältnis 89,2 / 10,8 angelagert wurde.

Tegostab® B2370:   Polyethersiloxan-basierender Schaumstoffstabilisator (Evonik Goldschmidt GmbH, Deutschland).

Addocat® 108:   Gemisch aminischer Katalysatoren für die Herstellung zu Polyurethan-Weichschaumstoff

Dabco® T-9 Catalyst:   Zinn(II)-Salz der 2-Ethylhexansäure

T80:   Gemisch aus 2,4- und 2,6-TDI im Gewichtsverhältnis 80 : 20 und mit einem NCO-Gehalt von 48 Gew.-%.

T65:   Gemisch aus 2,4- und 2,6-TDI im Gewichtsverhältnis 65 : 35 und mit einem NCO-Gehalt von 48 Gew.-%.

Unter den für die Herstellung von Polyurethanweichblockschaumstoffen üblichen Verarbeitungsbedingungen wurden die Ausgangskomponenten im Einstufenverfahren mittels Blockverschäumung verarbeitet. Die Kennzahl der Verarbeitung betrug in allen Fällen 108. Die Kennzahl gibt das Mengenverhältnis der Isocyanatgruppen in den Polyisocyanaten zu den gegenüber den Isocyanaten reaktiven Wasserstoffen in der PolyolFormulierung an. Der Kennzahl 108 entspricht ein Verhältnis von Isocyanatgruppen zu den gegenüber Isocyanaten reaktiven Wasserstoffen von 1.08:

$$\text{Kennzahl} = [(\text{Isocyanat-Menge eingesetzt}) : (\text{Isocyanat-Menge berechnet})] \bullet 100$$

Das Raumgewicht wurde bestimmt gemäß DIN EN ISO 845.

Die Stauchhärte (CLD 40 %) wurde bestimmt gemäß DIN EN ISO 3386-1-98 bei einer Verformung von 40 %, 4. Zyklus.

Die Zugfestigkeit wurde bestimmt gemäß DIN EN ISO 1798.

Der Druckverformungsrest (DVR 90 %) wurde bestimmt gemäß DIN EN ISO 1856-2000 bei 90 % Verformung.

**Tabelle 3:** Polyurethan-Weichblockschaumstoffe; Rezepturen und Eigenschaften

| Beispiel | | 11 | 12 (Vergleich) |
|---|---|---|---|
| Polyoletherpolyol 1 (aus Beispiel 1) | | 50 | - |

(fortgesetzt)

| Beispiel | | **11** | **12** (Vergleich) |
|---|---|---|---|
| Polyoletherpolyol 2 (aus Beispiel 2) | | - | 50 |
| Arcol® 1108 | | 50 | 50 |
| Wasser | | 3,50 | 3,50 |
| Tegostab® B 2370 | | 1,20 | 1,20 |
| Addocat® 108 | | 0,10 | 0,10 |
| Dabco® T9 Catalyst | | 0,16 | 0,16 |
| T80 | | 22,61 | 22,69 |
| T65 | | 22,61 | 22,69 |
| Zellstruktur | | fein | fein |
| Rohdichte | [kg/m$^3$] | 30,0 | 30,9 |
| Zugfestigkeit | [kPa] | 74 | 75 |
| Stauchhärte | [kPa] | 5,5 | 4,6 |
| DVR 90 % | [%] | 5,6 | 5,6 |

Die in der Tabelle 3 aufgeführten Ergebnisse zeigen, dass ein Weichschaumstoff hergestellt mit einer Mischung aus Polyetherpolyolen enthaltend ein nach dem erfindungsgemäßen Verfahren gefertigtes Polyetherpolyol eine signifikant höher Stauchhärte aufweist (Beispiel 11) als ein Weichschaumstoff, der ausschließlich auf Basis nicht erfindungsgemäßer Polyetherpolyole hergestellt wurde (Vergleichsbeispiel 12).

**Patentansprüche**

1. Verfahren für die Herstellung von Polyetherpolyolen (1) mit einer OH-Zahl von 3 mg KOH/g bis 150 mg KOH/g, **dadurch gekennzeichnet, dass**

   (i)

   (i-1) eine H-funktionelle Starterverbindung A1.1) mit einem oder mehreren Alkylenoxiden A 1.2) in Anwesenheit eines basischen Katalysators umgesetzt wird unter Erhalt eines Alkoxylates mit einer Äquivalentmolmasse von 53 Da bis 350 Da, und anschließend

   (i-2) die Komponente A1) mit Schwefelsäure neutralisiert wird, wobei die Neutralisation der alkalischen polymerisationsaktiven Zentren des rohen Alkylenoxidadditionsproduktes durch Zugabe von Schwefelsäure derart durchgeführt wird, dass von 66 mol-% bis 100 mol-% der eingesetzten Säure nur die erste Dissoziationsstufe zur Neutralisation der im Rohpolymerisat enthaltenden Katalysatormenge wirksam wird, und wobei auf die Abtrennung der gebildeten Salze verzichtet wird, unter Erhalt der Komponente A) und

   (ii) anschließend die Komponente A) umgesetzt wird mit einem oder mehreren Alkylenoxiden B1) in Anwesenheit eines DMC-Katalysators B2).

2. Verfahren gemäß Anspruch 1, **dadurch gekennzeichnet, dass** nach Schritt (i-2) in Schritt (i-3) die Entfernung von Reaktionswasser und mit der Säure eingebrachte Wasserspuren bei einem absoluten Druck von 1 bis 500 mbar und bei Temperaturen von 20 bis 200 °C erfolgt.

3. Verfahren gemäß Anspruch 1 oder 2, wobei als basischer Katalysator mindestens eine Verbindung ausgewählt aus der Gruppe bestehend aus Alkalimetallhydroxid, Erdalkalimetallhydroxid, Alkalimetallhydrid, Erdalkalimetallhydrid, Alkalimetallcarboxylat und Erdalkalimetallcarboxylat

4. Verfahren gemäß Anspruch 1 oder 2, wobei als basischer Katalysator Alkalimetallhydroxid verwendet wird.

**5.** Verfahren gemäß Anspruch 1 oder 2, wobei als basischer Katalysator Natriumhydroxid, Kaliumhydroxid und/oder Cäsiumhydroxid verwendet wird.

**6.** Verfahren gemäß einem der Ansprüche 1 bis 5, **dadurch gekennzeichnet, dass** die Konzentration an basischem Katalysator 40 ppm bis 5000 ppm, bezogen auf die resultierende Produktmenge A1), beträgt.

**7.** Verfahren gemäß einem der Ansprüche 1 oder 2, **dadurch gekennzeichnet, dass** als basischer Katalysator mindestens ein Amin verwendet wird.

**8.** Verfahren gemäß einem der Ansprüche 1 bis 7, **dadurch gekennzeichnet, dass** in Schritt (ii) ein Starterpolyol und DMC-Katalysator B2) im Reaktorsystem vorgelegt werden, und Komponente A) kontinuierlich gemeinsam mit einem oder mehreren Alkylenoxiden B1) zugeführt wird.

**9.** Verfahren gemäß Anspruch 8, **dadurch gekennzeichnet, dass** in Schritt (ii) als Starterpolyol eine Teilmenge an Komponente A) oder erfindungsgemäßes Endprodukt (1), das vorher separat hergestellt wurde, eingesetzt wird.

**10.** Verfahren gemäß einem der Ansprüche 1 bis 7, **dadurch gekennzeichnet, dass** in Schritt (ii) die gesamte Menge Komponente A) und DMC-Katalysator vorgelegt werden und eine oder mehrere H-funktionelle Starterverbindungen kontinuierlich gemeinsam mit einem oder mehreren Alkylenoxiden B1) zugeführt werden.

**11.** Verfahren gemäß einem der Ansprüche 1 bis 7, **dadurch gekennzeichnet, dass** in Schritt (ii) ein Starterpolyol und eine Teilmenge an DMC-Katalysator B2) im Reaktorsystem vorgelegt werden, und Komponente A) gemeinsam kontinuierlich mit einem oder mehreren Alkylenoxiden B1) und DMC-Katalysator zugeführt werden und das Polyetherpolyol (1) als Reaktionsprodukt kontinuierlich dem Reaktorsystem entnommen wird.

**12.** Verfahren gemäß Anspruch 11, **dadurch gekennzeichnet, dass** in Schritt (ii) als Starterpolyol eine Teilmenge an Komponente A) oder erfindungsgemäßes Endprodukt (1), das vorher separat hergestellt wurde, eingesetzt wird.

**13.** Verfahren nach einem der Ansprüche 1 bis 12, **dadurch gekennzeichnet, dass** die in Schritt (i-1) zu dosierenden Alkylenoxide A1.2) mindestens 10 % Ethylenoxid enthalten.

**14.** Polyetherpolyole erhältlich nach einem der Ansprüche 1 bis 13.

**15.** Polyurethane enthaltend Polyetherpolyole gemäß Anspruch 14.

**Claims**

**1.** A method for the preparation of polyether polyols (1) with a hydroxyl value of 3 mg KOH/g to 150 mg KOH/g, **characterised in that**

(i)

(i-1) an H-functional starter compound A1.1) is reacted with one or more alkylene oxides A1.2) in the presence of a basic catalyst, resulting in an alkoxylate with an equivalent molar mass of 53 Da to 350 Da, and then
(i-2) the component A1) is neutralised with sulfuric acid, the neutralisation of the alkaline, polymerisation-active centres of the crude alkylene oxide addition product being carried out by addition of sulfuric acid such that for from 66 mol % to 100 mol % of the acid used only the first dissociation step becomes effective for neutralisation of the amount of catalyst comprised in the crude polymer, and the separation of the salts formed being dispensed with, resulting in component A), and

(ii) then the component A) is reacted with one or more alkylene oxides B1) in the presence of a DMC catalyst B2).

**2.** A method according to Claim 1, **characterised in that** after step (i-2) in step (i-3) the removal of reaction water and traces of water introduced with the acid takes place at an absolute pressure of 1 to 500 mbar and at temperatures of 20 to 200°C.

3. A method according to Claim 1 or 2, with at least one compound selected from the group consisting of alkali metal hydroxide, alkaline-earth metal hydroxide, alkali metal hydride, alkaline-earth metal hydride, alkali metal carboxylate and alkaline-earth metal carboxylate as basic catalyst.

4. A method according to Claim 1 or 2, with alkali metal hydroxide being used as basic catalyst.

5. A method according to Claim 1 or 2, with sodium hydroxide, potassium hydroxide and/or caesium hydroxide being used as basic catalyst.

6. A method according to one of Claims 1 to 5, **characterised in that** the concentration of basic catalyst is 40 ppm to 5,000 ppm, relative to the resulting amount of product A1).

7. A method according to one of Claims 1 or 2, **characterised in that** at least one amine is used as basic catalyst.

8. A method according to one of Claims 1 to 7, **characterised in that** in step (ii) a starter polyol and DMC catalyst B2) are initially introduced into the reactor system, and component A) is supplied continuously together with one or more alkylene oxides B1).

9. A method according to Claim 8, **characterised in that** in step (ii) a partial amount of component A) or end product (1) according to the invention, which has been prepared separately beforehand, is used as starter polyol.

10. A method according to one of Claims 1 to 7, **characterised in that** in step (ii) the entire amount of component A) and DMC catalyst is initially introduced and one or more H-functional starter compounds are supplied continuously together with one or more alkylene oxides B1).

11. A method according to one of Claims 1 to 7, **characterised in that** in step (ii) a starter polyol and a partial amount of DMC catalyst B2) are initially introduced into the reactor system, and component A) is supplied jointly continuously with one or more alkylene oxides B1) and DMC catalyst, and the polyether polyol (1) is removed continuously from the reactor system as reaction product.

12. A method according to Claim 11, **characterised in that** in step (ii) a partial amount of component A) or end product (1) according to the invention, which has been prepared separately beforehand, is used as starter polyol.

13. A method according to one of Claims 1 to 12, **characterised in that** the alkylene oxides A1.2) to be metered in step (i-1) comprise at least 10% ethylene oxide.

14. Polyether polyols obtainable according to one of Claims 1 to 13.

15. Polyurethanes comprising polyether polyols according to Claim 14.

**Revendications**

1. Procédé de fabrication de polyéther-polyols (1) ayant un indice OH de 3 mg KOH/g à 150 mg KOH/g, **caractérisé en ce que**

   (i)

   (i-1) un composé de départ à fonction H A1.1) est mis en réaction avec un ou plusieurs oxydes d'alkylène A1.2) en présence d'un catalyseur basique pour obtenir un alcoxylate ayant une masse molaire équivalente de 53 Da à 350 Da, puis
   (i-2) le composant A1) est neutralisé avec de l'acide sulfurique, la neutralisation des centres alcalins actifs pour la polymérisation du produit d'addition d'oxyde d'alkylène brut étant réalisée par ajout d'acide sulfurique de sorte que seule la première étape de dissociation de 66 % en moles à 100 % en moles de l'acide utilisé agisse pour la neutralisation de la quantité de catalyseur contenue dans le polymère brut, et la séparation des sels formés étant omise, pour obtenir le composant A), puis

   (ii) le composant A) est mis en réaction avec un ou plusieurs oxydes d'alkylène B1) en présence d'un catalyseur DMC B2).

2. Procédé selon la revendication 1, **caractérisé en ce que** l'élimination de l'eau de réaction et des traces d'eau introduites avec l'acide a lieu après l'étape (i-2) à l'étape (i-3) à une pression absolue de 1 à 500 mbar et à des températures de 20 à 200 °C.

3. Procédé selon la revendication 1 ou 2, dans lequel au moins un composé choisi dans le groupe constitué par un hydroxyde de métal alcalin, un hydroxyde de métal alcalino-terreux, un hydrure de métal alcalin, un hydrure de métal alcalino-terreux, un carboxylate de métal alcalin et un carboxylate de métal alcalino-terreux est utilisé en tant que catalyseur basique.

4. Procédé selon la revendication 1 ou 2, dans lequel un hydroxyde de métal alcalin est utilisé en tant que catalyseur basique.

5. Procédé selon la revendication 1 ou 2, dans lequel de l'hydroxyde de sodium, de l'hydroxyde de potassium et/ou de l'hydroxyde de césium sont utilisés en tant que catalyseur basique.

6. Procédé selon l'une quelconque des revendications 1 à 5, **caractérisé en ce que** la concentration de catalyseur basique est de 40 ppm à 5 000 ppm, par rapport à la quantité de produit A1) résultante.

7. Procédé selon l'une quelconque des revendications 1 ou 2, **caractérisé en ce qu'**au moins une amine est utilisée en tant que catalyseur basique.

8. Procédé selon l'une quelconque des revendications 1 à 7, **caractérisé en ce qu'**un polyol de départ et un catalyseur DMC B2) sont chargés initialement dans le système de réacteur à l'étape (ii), et le composant A) est ajouté en continu conjointement avec un ou plusieurs oxydes d'alkylène B1).

9. Procédé selon la revendication 8, **caractérisé en ce qu'**une partie du composant A) ou d'un produit final selon l'invention (1), qui a été fabriqué séparément auparavant, est utilisée à l'étape (ii) en tant que polyol de départ.

10. Procédé selon l'une quelconque des revendications 1 à 7, **caractérisé en ce que** la totalité du composant A) et du catalyseur DMC sont chargés initialement à l'étape (ii), et un ou plusieurs composés de départ à fonction H sont ajoutés en continu conjointement avec un ou plusieurs oxydes d'alkylène B1).

11. Procédé selon l'une quelconque des revendications 1 à 7, **caractérisé en ce qu'**un polyol de départ et une partie du catalyseur DMC B2) sont chargés initialement dans le système de réacteur à l'étape (ii), et le composant A) est ajouté en continu conjointement avec un ou plusieurs oxydes d'alkylène B1) et le catalyseur DMC, et le polyétherpolyol (1) est extrait en continu du système de réacteur en tant que produit de réaction.

12. Procédé selon la revendication 11, **caractérisé en ce qu'**une partie du composant A) ou d'un produit final selon l'invention (1), qui a été fabriqué séparément auparavant, est utilisée à l'étape (ii) en tant que polyol de départ.

13. Procédé selon l'une quelconque des revendications 1 à 12, **caractérisé en ce que** les oxydes d'alkylène A1.2) à introduire à l'étape (i-1) contiennent au moins 10 % d'oxyde d'éthylène.

14. Polyéther-polyols pouvant être obtenus selon l'une quelconque des revendications 1 à 13.

15. Polyuréthanes contenant des polyéther-polyols selon la revendication 14.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5470813 A
- EP 700949 A
- EP 743093 A
- EP 761708 A
- WO 9740086 A
- WO 9816310 A
- WO 0047649 A
- WO 9729146 A
- WO 9803571 A
- EP 0090445 A
- EP 1400281 A
- EP 1577334 A
- WO 9914258 A
- US 6642423 B
- EP 1528073 A
- WO 2006094979 A
- WO 0153381 A
- EP 1073689 A
- EP 0855417 A
- WO 2007082596 A
- EP 1525244 A
- US 3404109 A
- US 3829505 A
- US 3941849 A
- US 5158922 A
- EP 10163170 A
- JP 4145123 A
- WO 0139883 A
- WO 0180994 A
- US 4987271 A
- DE 3132258 A
- EP 406440 A
- US 5391722 A
- US 5099075 A
- US 4721818 A
- US 4877906 A
- EP 385619 A
- US 3538043 A
- US 4500704 A
- US 5032671 A
- US 6646100 A
- EP 222453 A
- WO 2008013731 A
- US 6013596 A

**In der Beschreibung aufgeführte Nicht-Patentliteratur**

- **VON M. IONESCU et al.** *Advances in Urethanes Science and Technology,* 1998, vol. 14, 151-218
- Ullmann's Encyclopedia of Industrial Chemistry. 1992, vol. B4, 167ff
- B. Handbuch Apparate. Vulkan-Verlag Essen, 1990, 188-208